# EUROPEAN PATENT APPLICATION

(11) **EP 4 699 628 A1**
(43) Date of publication of application: **25.02.2026**
(21) Application number: 25196640.4
(22) Date of filing: 19.08.2025
(51) Int. Cl.: A61M 1/36, A61M 1/34

(54) **CELL SELECTION CHAMBER AND METHODS OF CELL SELECTION**

(30) Priority: 21.08.2024 US 202463685398 P; 21.08.2024 US 202463685693 P
(71) Applicant: Fenwal, Inc., Lake Zurich, IL 60047 (US)
(72) Inventor: THOMPSON, Kyle, Lake Zurich, 60047 (US); WEGENER, Christopher J., Lake Zurich, 60047 (US)
(74) Representative: Maikowski & Ninnemann Patentanwälte Partnerschaft mbB

(57) **Abstract**

A cell selection chamber includes an inlet and an outlet, with a reservoir positioned therebetween and configured to allow for fluid flow from the inlet to the outlet. The reservoir contains a plurality of microbubbles, which are each at least partially coated with streptavidin. An upstream membrane is positioned between the inlet and the reservoir, while a downstream membrane is positioned between the outlet and the reservoir. The membranes include a plurality of pores each having a diameter that is less than a diameter of the microbubbles. In use, a heterogeneous population of cells is mixed with a biotinylated antibody additive and then conveyed into the cell selection chamber so as to cause target cells of the heterogeneous population of cells to become bound to the microbubbles and to cause other cells of the heterogeneous population of cells to flow out of the cell selection chamber as unbound cells.

## Description

### Background

### Field of the Disclosure

The present disclosure relates to selection or separation of target cells from a heterogeneous population of cells. More particularly, the present disclosure relates to selection or separation of target cells from a heterogeneous population of cells using streptavidin-coated microbubbles contained within a cell selection chamber.

### Description of Related Art

Various blood processing systems make it possible to separate blood into two or more constituent parts, which may be useful for donation purposes and for treatment of individuals with potentially detrimental or harmful conditions or disorders.

When such systems are used for blood component donation, whole blood is typically drawn from a donor, a particular blood component or constituent is removed and collected, and the remaining blood constituents are returned to the donor.

Such systems can also be used to provide blood components for use in cellular therapies for patients. For these therapies it is typical to separate a particular cellular or other blood component from whole blood and modify, enrich and/or expand the collected component before returning it to the patient as part of a therapeutic treatment. For example, one such therapy, Chimeric antigen receptor (CAR) T-cell therapy, alters a patient's T-cells and adds an artificial receptor to the cells that attach to cancer cell antigens. These modified T-cells are returned to the patient and can help target and destroy specific cancer cells.

In order to modify the T-cells, they must first be isolated from other cells. Various approaches for separating certain blood cells (including T-cells) from other cellular blood components are known, including buoyant separation (as described, for example, in U.S. Patent Application Publication Nos. 2016/0167061 and 2020/0009614, which are hereby incorporated herein by reference) and chromatographic isolation (as described, for example, in U.S. Patent Application Publication No. 2019/0226951, which is hereby incorporated herein by reference). Other known approaches entail a target cell population being bound to small, magnetic particles (thus requiring the use of a strong magnet for cell isolation) or use of large amounts of contaminants (thus requiring centrifugation or spinning membrane separation).

### Summary

There are several aspects of the present subject matter which may be embodied separately or together in the devices and methods described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a cell selection chamber includes an inlet, an outlet, and a reservoir positioned between the inlet and the outlet. The reservoir is configured to allow for fluid flow from the inlet to the outlet and contains a plurality of microbubbles that are each at least partially coated with streptavidin. An upstream membrane is positioned between the inlet and the reservoir, while a downstream membrane is positioned between the outlet and the reservoir, with the membranes including a plurality of pores each having a diameter less than a diameter of the microbubbles.

In another aspect, a blood processing system includes a blood processing device and a fluid flow circuit that is configured to be mounted to the blood processing device. The blood processing device includes a pump system, a valve system, and a controller, while the fluid flow circuit includes a cell selection chamber having an inlet, an outlet, and a reservoir positioned between the inlet and the outlet. The reservoir is configured to allow for fluid flow from the inlet to the outlet and contains a plurality of microbubbles that are each at least partially coated with streptavidin. An upstream membrane is positioned between the inlet and the reservoir, while a downstream membrane is positioned between the outlet and the reservoir, with the membranes including a plurality of pores each having a diameter less than a diameter of the microbubbles. The controller is programmed to control the pump system and the valve system during a blood processing procedure to convey a heterogeneous population of cells into the cell selection chamber so as to cause target cells of the heterogeneous population of cells to become bound to the microbubbles and to cause other cells of the heterogeneous population of cells to flow out of the cell selection chamber as unbound cells.

In yet another aspect, a method is provided for selecting target cells from a heterogeneous population of cells. The method includes mixing a heterogeneous population of cells with a biotinylated antibody additive including regions configured to bind to the target cells and not to other cells of the heterogeneous population of cells. The heterogeneous population of cells is conveyed into a cell selection chamber via an inlet of the cell selection chamber, with the heterogeneous population of cells flowing through an upstream membrane and into a reservoir of the cell selection chamber containing a plurality of microbubbles each at least partially coated with streptavidin so as to cause the target cells to become bound to the microbubbles. The other cells flow through a downstream membrane, out of the reservoir, and out of the cell selection chamber as unbound cells via an outlet of the cell selection chamber.

### Brief Description of the Drawings

Fig. 1 is a schematic view of an exemplary fluid flow circuit;
Fig. 2 is a schematic view of an exemplary blood processing device;
Fig. 3A is a perspective view of the fluid flow circuit of Fig. 1;
Fig. 3B is a perspective view of the blood processing device of Fig. 2, in an open condition, with an inserted fluid flow circuit according to Fig. 1;
Fig. 3C is a perspective view of a blood processing system comprising the blood processing device of Fig. 2 (in a closed condition) and the fluid flow circuit of Fig. 1;
Fig. 4 is a front perspective view of the fluid flow circuit of Fig. 1, shown in greater detail;
Fig. 5 is a rear perspective view of the fluid flow circuit of Fig. 1;
Fig. 6 is a schematic view of a first or user-adjacent portion of a fluid flow circuit according to the present disclosure associated to a portion of a blood processing device according to the present disclosure;
Fig. 7 is a schematic view of a first or user-adjacent portion of another embodiment of a disposable fluid flow circuit according to the present disclosure associated to a portion of a blood processing device according to the present disclosure;
Fig. 8 is a schematic view of an exemplary second portion of a disposable fluid flow circuit according to the present disclosure;
Fig. 9 is a schematic view of a priming step of a blood processing procedure using the disposable fluid flow circuit of Fig. 8;
Fig. 10 is a schematic view of a first separation step of a blood processing procedure using the disposable fluid flow circuit of Fig. 8;
Fig. 11 is a schematic view of a second separation step of a blood processing procedure using the disposable fluid flow circuit of Fig. 8;
Fig. 12 is a schematic view of a cell concentration step of a blood processing procedure using the disposable fluid flow circuit of Fig. 8;
Fig. 13 is a schematic view of a first cell formulation step of a blood processing procedure using the disposable fluid flow circuit of Fig. 8;
Fig. 14 is a schematic view of a second cell formulation step of a blood processing procedure using the disposable fluid flow circuit of Fig. 8;
Fig. 15 is a schematic view of a delivery step of a blood processing procedure using the disposable fluid flow circuit of Fig. 8;
Fig. 16 is a schematic view of a cell selection step of a blood processing procedure using the disposable fluid flow circuit of Fig. 8;
Fig. 16A is a schematic view of an exemplary cell selection chamber used during the cell selection step of Fig. 16;
Fig. 16B is a schematic view of a homogeneous population of cells being mixed with a biotinylated antibody additive during the cell selection step of Fig. 16;
Fig. 16C is a schematic view of the mixture of Fig. 16B being conveyed into the cell selection chamber of Fig. 16A during the cell selection step of Fig. 16;
Figs. 17-19 are schematic views of exemplary biological cell processing systems according to the present disclosure;
Fig. 20 is a schematic view of an exemplary pressure control system of one embodiment of a biological cell processing system according to the present disclosure; and
Fig. 21 and 22 are schematic views of exemplary fluid flow paths of biological cell processing systems according to the present disclosure.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

The disclosure will be more fully understood from the following description taken in conjunction with the accompanying drawings. Some of the figures may have been simplified for the purpose of more clearly showing other elements. Such simplification of the figures is not necessarily indicative of the presence or absence of particular elements in any of the exemplary embodiments, except as may be explicitly delineated in the corresponding written description. The drawings are not necessarily to scale.

The current disclosure includes exemplary embodiments of fluid flow circuits and blood processing devices, which are combinable to form an automated blood processing system for collecting, separating, concentrating, and modifying blood cells for reinfusion back into a patient. Also described herein are exemplary cell selection chambers and methods for using such chambers, though it should be understood that such cell selection chambers are not limited to use in fluid flow circuits of the type described herein, use in combination with blood processing devices of the type described herein, or methods of the type described herein, but may be practiced in combination with other fluid flow circuits, blood processing devices, and blood processing methods.

As used herein, the term "blood" includes without limitation blood and blood components, and the terms "cell" or "biological cell" include without limitation blood cells, such as red cells, white blood cells, and T-cells. By "automated," it is meant that the apparatus can be programmed to carry out the processing steps of a biological fluid processing method without substantial operator involvement. Of course, even in the automated system of the present disclosure, it will be understood that some operator activity may be involved, including the loading of the disposable fluid circuits and entering processing parameters. Additional manual steps may be required as well. However, the reusable apparatus can process blood through the disposable circuit(s) described below without substantial operator intervention.

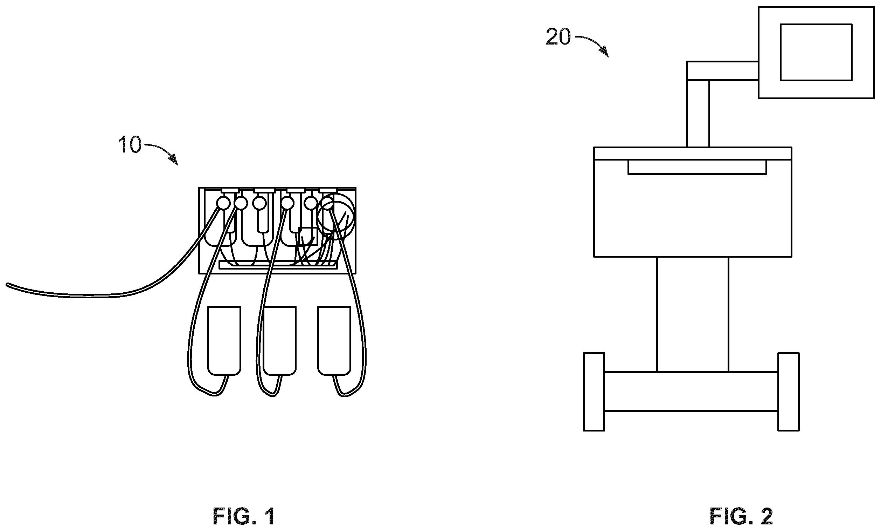

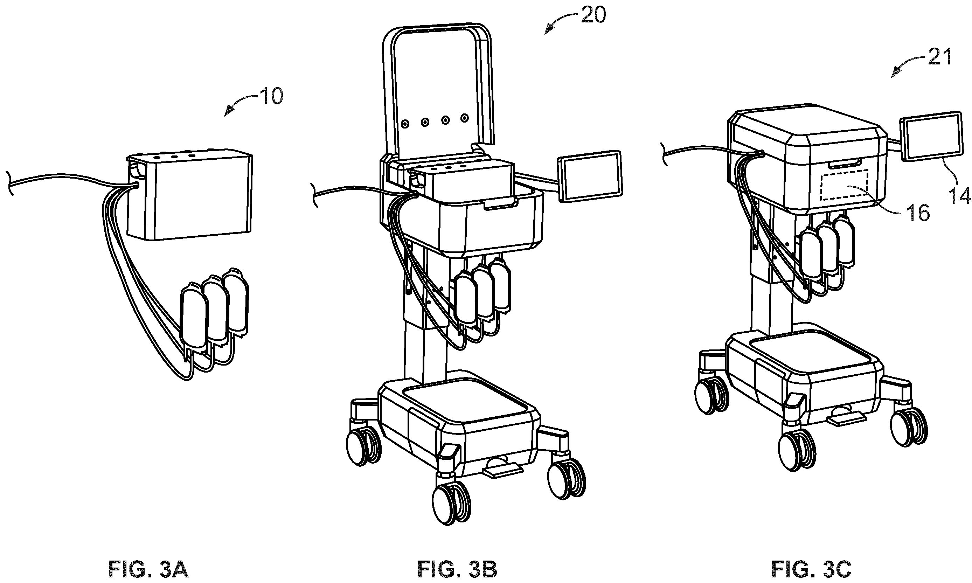

An exemplary blood processing system 21 (Fig. 3C) according to the present disclosure includes two principal components - a durable and reusable blood or cell processing device 20 (Figs. 2 and 3B) and a disposable fluid flow circuit 10 (Figs. 1 and 3A). The blood processing device 20 includes components that control and monitor fluid flow through the disposable flow circuit 10 and a controller 16 (Fig. 3C), which is programmed to govern and/or direct the operation of the other components of the blood processing device 20 to perform a blood processing procedure selected by the operator, as will be described in greater detail.

Blood processing systems and methods according to the current disclosure are described as utilizing a blood processing device or system and different cell separating, concentrating and modifying modules. However, it should be understood that the principles described herein are not limited to a particularly configured device and/or a particular sequence of steps or stages. Rather, the blood processing systems and methods described herein may be applied using a variety of differently configured blood processing devices and fluid flow circuits that carry out blood processing procedures in different ways.

The blood processing device 20 (as shown in Figs. 2, 3B, and 3C) can be modular and designed with a plurality of components that work with various fluid flow circuits 10 to process blood components. The blood processing device 20 may include movable or mobile components, such as wheels, for moving the device 20 to and from a patient's bed or chair. The device 20 may be placed at least substantially or even completely at the patient's point-of-care (e.g., bedside or chairside). The device 20 can accommodate all manufacturing unit operations in a single system, as will be described below. The blood processing device 20 may include valves or valve components (which are collectively referred to herein as a "valve system"), air control systems, pumps (which are collectively referred to herein as a "pump system"), detectors, sensors, one or more controllers, a user interface, and any other components that may function in combination to aid with moving fluid through the fluid flow circuit 10.

The blood processing device 20 may include valves or motors associated with valve portions of the fluid flow circuit 10. The valves may be configured to interact with the conduits of a fluid flow circuit 10 mounted to the device 20. As examples only, the valves can be solenoid pinch valves, motor-driven rotary pinch valves, linear actuators, stop cocks or any other type of automated clamping or valve device known in the art. In an exemplary embodiment, the blood processing device 20 includes valve motors compatible with valve components on the fluid flow circuit 10.

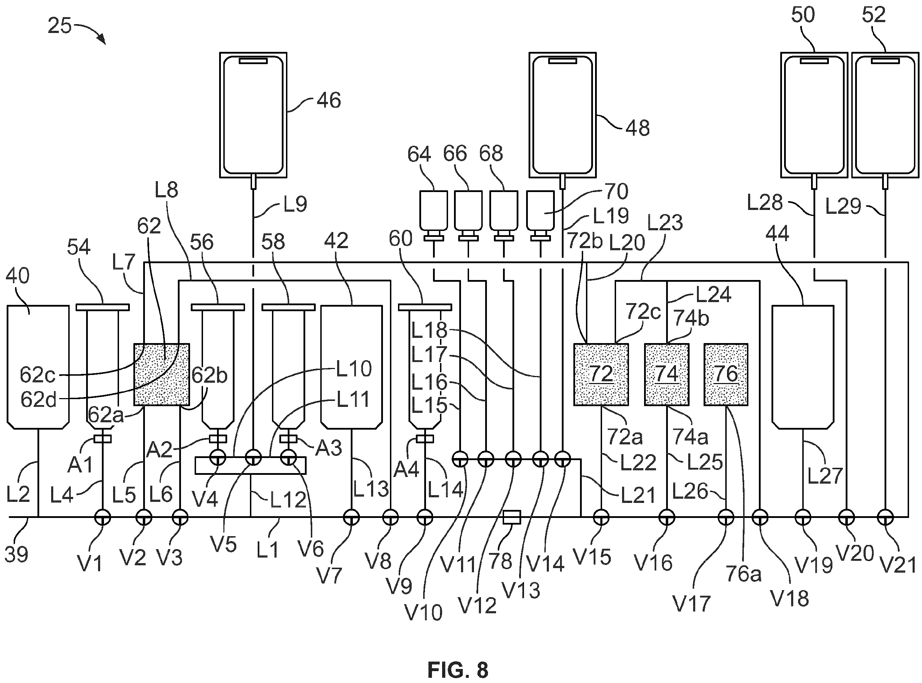

The blood processing device 20 may also include air control systems for providing air to, for example, pneumatic syringe pump assemblies (which are identified in Figs. 8-16 at 54, 56, 58, and 60), discussed further below. The air control system may include a vacuum and/or pressure source, such as a diaphragm pump. The vacuum or pressure source may pump filtered air in and out of the pump. Examples and further details of assemblies that may be utilized in the blood processing device 20 are described in U.S. Patent Nos. 10,926,895; 11,191,880; and 10,781,001; and 11,827,398, each of which is hereby incorporated herein by reference.

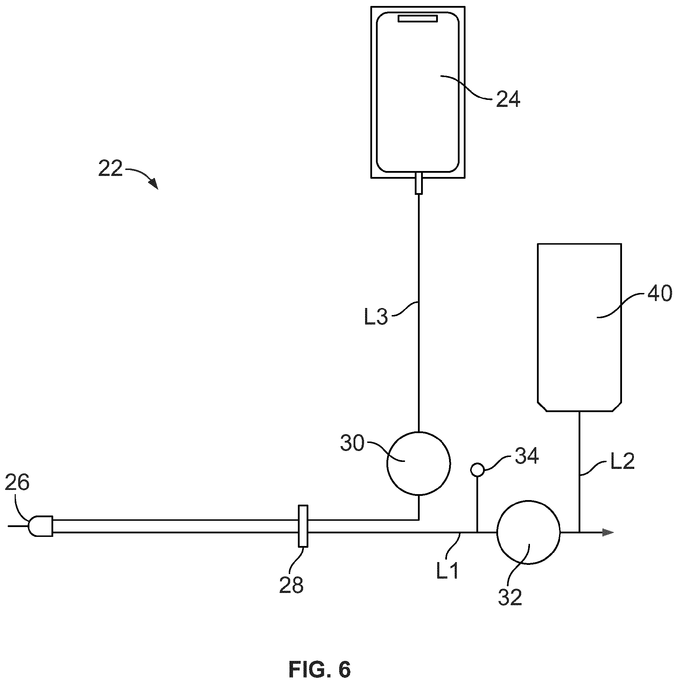

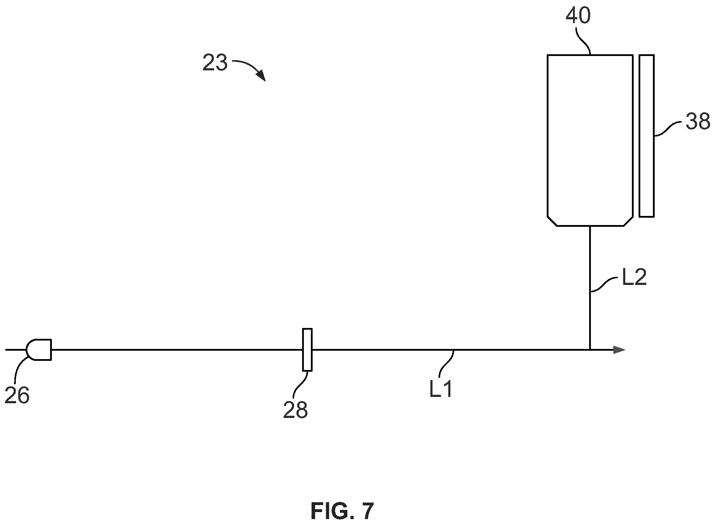

The blood processing device 20 may also include one or more pumps (with Fig. 6 illustrating two possible pumps 30 and 32) that may be associated with a user-adjacent portion of a fluid flow circuit 10 to initiate and cause fluid to flow through the circuit 10. If a user-adjacent portion is not utilized or a passive user-adjacent portion (such as shown in Fig. 7) is utilized, the blood processing device 20 does not need to include pumps of the type shown in Fig. 6. If provided, the pumps 30 and 32 may be differently or similarly configured and/or function similarly or differently from each other. In an exemplary embodiment, the pumps are configured as peristaltic pumps, which may be generally configured as described in U.S. Patent No. 5,868,696, which is hereby incorporated herein by reference. Each pump 30, 32 may engage a different line of the fluid flow circuit 10 and may be selectively operated under command of the controller 16 to cause fluid to flow through a portion of the fluid flow circuit 10, when using a user-adjacent portion of a fluid flow circuit 10 of the type shown in Fig. 6.

The illustrated blood processing device 20 can also include air detectors, which are identified in Fig. 8 as A1 - A4 (and configured, for example, as ultrasonic bubble detectors) and which accommodate tubing of the fluid flow circuit 10 that flows fluid to a recipient. It may be advantageous to prevent air from reaching the recipient, whether a human recipient (e.g., the same human that serves as the blood source) or a non-human recipient (e.g., a storage bag or container), so the air detectors A1 - A4 may transmit signals to the controller 16 that are indicative of the presence or absence of air in the tubing. If the signal is indicative of air being present in the tubing, the controller 16 may initiate an alarm or error condition to alert an operator to the condition and/or to take corrective action to prevent the air from reaching the recipient (e.g., by reversing the flow of fluid through the tubing or diverting flow to a vent). The air detectors A1 - A4 may alternatively or additionally be used as part of a fluid flow control system for the fluid flow circuit 10.

The illustrated blood processing device 20 may also include one or more sensors or sensing elements for sensing a condition or characteristic of a blood component. For example, in one embodiment, a cell density sensor 78 (such as shown in Figs. 8-16 on line L1) may be incorporated to detect absolute or relative changes in cell concentration. Optical devices that use methods such as light transmission, scatter, or spectroscopy could be used. Devices that utilize electrical methods such as capacitance could also be useful. Even devices that use acoustic methods could yield relational density measurements.

Also, when the system is provided with pumps configured as syringe pumps 54, 56, 58, 60 (as shown in Figs. 8-16 and described below), the plunger position of any of such pumps may be tracked by a sensing element. Additionally, pressure sensors may be incorporated into the system to monitor the pressure at various locations of the fluid flow circuit 10. For instance, if the blood source is a human donor, one or more pressure sensors, such as donor pressure sensor 34 (Fig. 6) may be configured to monitor the pressure of the donor's vein during blood draw and return. The controller 16 may receive signals from the pressure sensor 34 that are indicative of the pressure within the fluid flow circuit 10 and, if a signal indicates a low- or high-pressure condition, the controller 16 may initiate an alarm or error condition to alert an operator to the condition and/or to attempt to bring the pressure to an acceptable level without operator intervention.

As noted above, the blood processing device 20 includes a controller, such as the controller 16 shown in Fig. 3C. Although shown in the upper part of the blood processing device 20, the controller may be incorporated into different parts of the blood processing device 20. The controller may, according to the embodiments described herein, include a programmable microprocessor, which microprocessor may be programmed to operate the blood processing device 20 and system 21 according to a process.

According to other embodiments, the controller may include one or more electrical circuits designed to carry out the actions described herein. In addition, the controller may include one or more memories. The instructions by which the microprocessor is programmed may be stored on the memory associated with the microprocessor, which memory/memories may include one or more tangible non-transitory computer readable memories, having computer executable instructions stored thereon, which when executed by the microprocessor, may cause the microprocessors to carry out one or more actions as described below.

The controller may be coupled to one or more of the structures of the blood processing device 20 and also structures of the fluid flow circuit 10, for example to receive information (e.g., in the form of signals) from these structures or to provide commands (e.g., in the form of signals) to these structures to control the operation of the structures. The controller may be coupled to the sensors, valves, and pumps to provide commands to those devices to control their operation. It may also be possible that the controller receives information from and provides commands to a given structure, such as one of the structures already mentioned. The controller may be directly electrically connected to these structures to be coupled to them, or the controller may be directly connected to other intermediate equipment that is directly connected to these structures to be coupled to them.

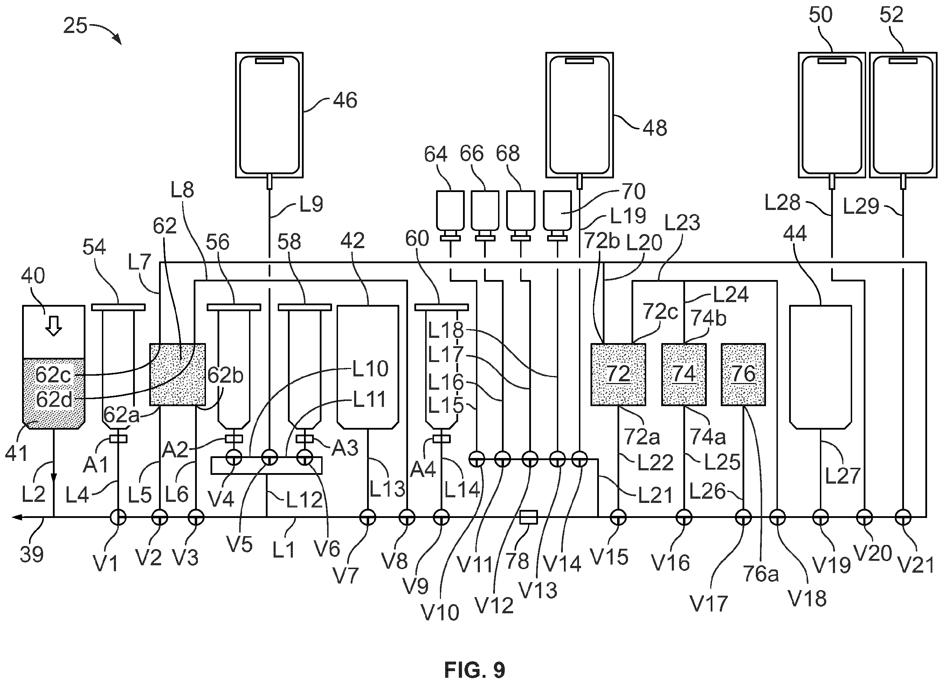

The controller is configured and/or programmed to execute at least one blood processing procedure (with steps of an exemplary procedure being shown in Figs. 9-16 and described below) but, more advantageously, is configured and/or programmed to execute multiple types of blood processing procedures which may include a blood separating phase, a cell selection phase, and a cell modifying phase.

More particularly, in carrying out any blood processing procedure, the controller is configured and/or programmed to control the amount of a fluid that flows from one component to another and the flow rate of such fluid. This may include instructing the valves to open and close at specific points during a procedure or initiating transfer of a fluid from one container to another. Hence, while it may be described herein that a particular component of the blood processing system performs a particular function, it should be understood that that component is being controlled by the controller to initiate and/or perform that function.

A user interface screen 14 (e.g., a touchscreen) may be associated with the blood processing device 20 (as in Fig. 3C). The user interface screen 14 may allow an operator to interact with the system controller 16 (e.g., a microprocessor) of the device 20 to provide instructions to the controller (e.g., to carry out a particular procedure), as well as providing information to the controller to be used during a procedure (e.g., white blood cell ("WBC") pre-count of the blood of the blood source). The user interface screen 14 may serve as a display to provide the operator with instructions (e.g., to connect or disconnect the blood source from the flow circuit 10) and information (e.g., alerting the operator to a blockage in a fluid flow conduit of the flow circuit 10), including providing a procedure status.

The blood processing device 20 may include computer equipment that permits the blood processing device 20 (including the controller 16) to communicate (whether via wires, cables, etc. or wirelessly) with other blood processing devices over a local network, or with other blood processing devices or other computer equipment (e.g., a server) over local networks, wide area networks, or the Internet. According to such an embodiment, the blood processing device 20 may include an internal transmitter/receiver device.

Figs 3A-3C show insertion and loading steps of a fluid flow circuit 10 into the blood processing device 20 to provide a single, modular blood processing system 21. The fluid flow circuit 10 (Fig. 3A) is inserted into an open cabinet of the blood processing device 20 (Fig. 3B) and the components are connected as needed before closing the cabinet in a third step (Fig. 3C). The blood processing system 21, including the blood processing device 20 and fluid flow circuit 10 is a single modular system, which is capable of performing the entirety of the blood processing procedure without movement to outside components or devices.

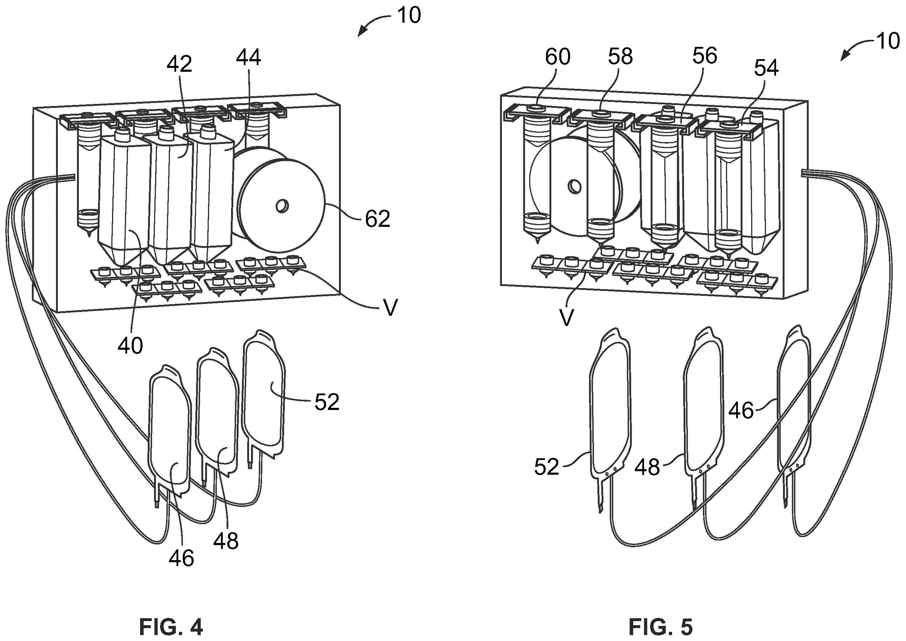

As for the fluid flow circuit or flow set 10 (which is shown in greater detail in Figs. 4-5 and 8), it is intended to be a sterile, single use, disposable item. Figs. 4 and 5 include perspective views of the fluid flow circuit 10, and Fig. 8 is a schematic illustration of the fluid flow circuit 10. Figs. 9-16 show different stages of an exemplary blood processing procedure. The illustrated fluid flow circuit 10 is modular, and different customizable configurations of the fluid flow circuit 10 can be loaded into a blood processing device 20. Before beginning a given blood processing and modifying procedure, the operator mounts the fluid flow circuit 10 to the blood (cell) processing device 20. The controller 16 implements the procedure based upon preset protocols, taking into account other input from the operator. Upon completing the procedure, the operator removes the fluid flow circuit 10 from association with the blood processing device 20. If any portions of the fluid flow circuit 10 are holding modified blood components (such as doses) at the end of a procedure, such portions are removed from the device 20 and retained for storage, transfusion, or further processing. The remainder of the fluid flow circuit 10 is removed from the blood processing device 20 and discarded.

The different fluid flow circuits used in combination with the blood processing device may vary slightly in components depending on the blood or cell processing procedure to be carried out using the system and the resulting products to be produced. Accordingly, different fluid flow circuits may be used in connection with particular blood processing procedures. The fluid circuit is completely customizable, and as such, various components can be added and removed. Generally speaking, the fluid flow circuit 10 may include pumps, reservoirs, valve components, fluid input and output containers, a separation device, a cell selection chamber (which will be described in greater detail herein), a concentration device, and at least one cell modifying module (as shown in Fig. 8) or combinations thereof.

The fluid flow circuit may include two different portions, a first or user-adjacent portion (22 or 23, shown in Figs. 6 and 7), used when connecting the system to a blood source and a second or processing portion 25 (shown in Fig. 8). The user-adjacent portion can be selected based on whether the process will be active (reinfusion back to the blood source) or passive (in which a dose is collected for later infusion). Fig. 6 shows a first possible user-adjacent portion 22 with components and a schematic of an active process. Fig. 7 shows a second possible user-adjacent portion 23 with components and a schematic of a passive process.

Both user-adjacent portions include at least one blood source access device 26 (e.g., a phlebotomy needle), used to draw blood from a blood source and (optionally) convey fluid to the blood source. In one embodiment, two blood source access devices (e.g., two phlebotomy needles) may be used, with one serving to draw blood into the flow circuit 10 from a source and the other being used to return fluid to the source. In another embodiment, a blood source (such as a previously collected bag) may be attached to the system. Both user-adjacent portions are shown as including a donor isolation clamp 28. Main line L1 is present in both illustrated user-adjacent portions as well, connecting the fluid processing portion 25 (Fig. 8) of the fluid flow circuit 10 to the user adjacent portion 22 or 23.

In the "active" embodiment of the user-adjacent portion 22 shown in Fig. 6, two separate flow lines connect to the blood source access device 26 - a first line L3 connected to an anticoagulant container 24 and a second line L2 connected to a reservoir 40. The blood processing device 20 may include an associated anticoagulant pump 30 and a draw/return pump 32 for moving fluid to and from a blood source.

The "passive" user-adjacent portion 23 shown in Fig. 7 only requires connection to the reservoir 40 and, optionally, a level-sensing element 38 and, therefore, only includes the main line L1. The anticoagulant may be added to reservoir 40 before processing.

If the blood processing device 20 is provided with pumps configured to act upon the user-adjacent portion of a fluid flow circuit 10, the user-adjacent portion of the fluid flow circuit 10 may include appropriate formations that are mated to the pumps. For example, Fig. 6 shows two pumps 30 and 32 that are actuated by the controller 16 to cause fluid to flow through the fluid flow circuit 10. In an exemplary embodiment, the pumps are configured as peristaltic pumps, in which case the associated portions of the fluid flow circuit 10 may be configured as flexible tubing lines that are engaged and manipulated by the pumps 30 and 32 to cause fluid to flow through a portion of the fluid flow circuit 10. In another exemplary embodiment, the fluid flow circuit 10 may instead include a defined pump chamber that is acted upon by a reciprocating pump actuator of the blood processing device 20 to selectively convey fluid through a portion of the circuit 10. Indeed, it should be understood that any suitable pump system may be employed without departing from the scope of the present disclosure.

Turning now to the second or fluid processing portion 25 of the fluid flow circuit 10, an exemplary configuration is shown in Fig. 8. The fluid flow circuit 10 may include a plurality of fluid input and output containers. Each container may be integrally formed with the fluid flow circuit 10 or they may be connected to the fluid flow circuit (e.g., by piercing a septum of a tube of the fluid flow circuit, via a luer connector, or by sterilely joining using a sterile welding system) before the fluid flow circuit is mounted to the blood processing device, forming the blood processing system 21. The containers may be composed of any desired medical grade material, such as medical grade plastic. The fluid input containers included in the processing portion 25 of the fluid flow circuit may include at least one buffer container 46 and at least one solution or liquid container 64. The solution or liquid containers may be configured to hold a liquid chemical composition for mixing with blood or blood cell components. Fig. 8 shows optional first buffer container 46 and second buffer container 48 and, optionally, four solution or liquid containers 64, 66, 68, and 70. However, the amount of buffer containers and the amount and/or presence of solution or liquid containers may vary based on the cell modification process being utilized.

Output containers may also be integrally formed with the fluid flow circuit 10 or may be connected to the fluid flow circuit 10. These containers are meant to house different cellular fractions, spent buffers, formulated suspensions, or accommodate samples thereof. These containers may include a waste container 52 and final dose container 50, which are both shown in Fig. 8. In a process where a dose of modified cells is not immediately returned to the blood source after modification, the dose container 50 may be a removable container which is transferred after the procedure.

The fluid flow circuit may include valves or valve arrays (V1-V21 as shown in Figs. 8-16). The valves may be stopcock valves. These valves cooperate with/interface with motors that are part of the blood processing device hardware. The valves may be used to direct flow between different elements of the fluid flow circuit. Other types of valves (such as solenoid-driven valves, which are described below) may be employed without departing from the scope of the present disclosure.

The fluid flow circuit may also include pumps 54, 56, 58, 60. Although four pumps are shown in Fig. 8, the fluid flow circuit may utilize more or fewer pumps. The pumps are preferably pneumatic syringe pump assemblies that interface with air controls systems of the blood processing device 20 hardware. Positive or negative pressure can be applied to displace the syringe plunger. Positive pressure translates to fluid flow out of the pump, with negative pressure translating to fluid flow into the pump. Optionally, there may be a sterile filter embedded with a cap of the syringe. In an exemplary embodiment, the pumps may be generally configured as described in U.S. Patent Application Publication No. 2021/0121827, which is hereby incorporated herein by reference in its entirety. As noted above, the plunger positions may be tracked by a sensing element on the hardware. Pumps can be operated in pressure-targeting mode or in flow-rate targeting mode, depending on the control scheme required for that processing step. The pumps may also be configured as pneumatic syringe pumps of the type described in U.S. Provisional Patent Application No. 63/615,004, filed December 27, 2023, and hereby incorporated herein by reference.

The fluid flow circuit may include a plurality of reservoirs 40, 42, and 44. Such reservoirs, if provided, may be used as passive vessels to hold fluids before, during, and after processing steps. The reservoirs may be vented with sterilizing filters such that flow into or out of the reservoir does not result in pressurization of the vessel. Although Fig. 8 shows a fluid flow circuit with three reservoirs, more or fewer may be included in the fluid flow circuit 10.

The reusable hardware processing device may include at least one weigh scale associated with at least one of the containers of the fluid flow circuit. There may be a weigh scale associated with a first buffer container 46, a second buffer container 48, one or more of the four solution or liquid containers 64, 66, 68, and 70, any of the reservoirs 40, 42, and 44, the dose or sample container 50, and the waste container 52. Any of the containers of the circuit configured to hold fluid for a time may be associated with a weigh scale of the blood processing device 20 to monitor the amount of liquid added or removed.

The fluid flow circuit 10 may include a separation module 62 (Fig. 8). In one embodiment, which will be described in more detail below, a microfluidic separation module 62 may be employed to continuously separate particles or cells. The microfluidic separation module may include a plurality of channels used for separating by cell characteristic, such as diameter. For instance, the critical diameter of ~7um could separate nucleated white blood cells from red blood cells and platelets. The separator module 62 shown in Fig. 8 includes two outputs 62c and 62d, with a first output 62d for cells larger than a designated critical diameter and a second output 62c for cells smaller than a designated critical diameter. These separator modules can also be used to displace target cell populations into new buffers, effectively "washing" said cell suspensions. In alternate embodiments, the separator module may include a spinning membrane separator or centrifugal separation chamber utilized in other blood processing devices, such as those described in greater detail in one or more of U.S. Patent Nos. 4,526,515; 5,194,145; 6,312,607; 6,524,231; 4,094,461; 7,052,606; 4,300,717; and 8,075,468, all of which are hereby incorporated herein by reference. If a spinning membrane separator or centrifugal separator are utilized, the blood processing device may include the applicable hardware components.

The fluid flow circuit 10 may include a concentrator module or microfluidic concentrator module 72 that may be employed to continuously concentrate particles or cells, generating a concentrated output stream at outlet 72c and a supernatant output stream at outlet 72b from a single input stream at inlet 72a as shown in Fig. 8. The concentrator module 72 may operate at a fixed concentration rate (for instance 10x) per pass through the module. Desired cell concentrations can be targeted by performing series of fixed concentrations and dilution steps. In one example, white blood cells may be concentrated 25x. Alternatively, the concentrator module may achieve a variable concentration by passing through the module multiple times and including a dilution between passes. As an example, a concentration such as 15x can be achieved by this method.

The fluid flow circuit 10 may be further configured to interface with or include at least one cell modification module. In one example, these modules can perform cell and/or gene therapy. Accordingly, the fluid flow circuit 10 may be configured to interface with one or more of a delivery module and a cell selection module.

A delivery module 74 may also be part of the fluid flow circuit 10 as shown in Fig. 8. In one embodiment, the delivery module may be a gene delivery module in which a cell is genetically modified. The module may be employed to administer intracellular payloads and cell suspension. The delivery module 74 can include an inlet 74a and an outlet 74b. Examples of modules may be electroporators, mechanoporators, sonoporators, soluporators or other flow-through transfection technologies and/or means of introducing therapeutic payloads. In an exemplary embodiment, the delivery module may include an electroporation device of the type disclosed in U.S. Patent Application Publication No. 2020/0282116, which is hereby incorporated herein by reference.

A cell selection module 76, such as an affinity-based cell selection module may also be part of the fluid flow circuit 10, as shown in Fig. 8. The cell selection module 76 may be employed to phenotypically isolate a target cell population from a bulk cell suspension. In the embodiment shown in Fig. 8, the cell selection module 76 includes an inlet 76a which may also function as an outlet, though other embodiments employ a cell selection module having a distinct inlet and outlet (as will be described in greater detail herein). Technologies such as magnetic-beads, affinity chromatography, or filtration could be implemented using positive or negative selection methods. In one embodiment, the cell selection module may include an affinity column of the type described in U.S. Provisional Patent Application No. 63/613,500, filed December 21, 2023, and hereby incorporated herein by reference. In another embodiment, which will be described in greater detail below and which may be used in combination with or instead of a separation module 62 employing one of the aforementioned technologies, the cell selection module 76 may be configured as or include a cell selection chamber containing a plurality of streptavidin-coated microbubbles.

According to another aspect of the present disclosure, the fluid flow circuit 10 may utilize existing portions of the fluid flow circuit 10 as a cell modification module such as for cell formulation. In such embodiments, a solution or liquid chemical additive may be added to blood cells in a reservoir (such as one of reservoirs 42 or 44) that is integrated with the fluid flow circuit or a pump (such as pump 60) or both. The blood cells may formulate, mix, or incubate in this reservoir/pump or be moved between components of the fluid flow circuit 10.

As noted, the various components of the fluid flow circuit 10 may be connected by flexible tubing or any other suitable fluid flow conduit. In one embodiment, the fluid flow circuit may include a cassette. The cassette may be rigid or flexible. The illustrated fluid flow circuit 10 includes lines L1-L32 (shown in detail in Fig. 8), though lines may be added or subtracted depending on the desired configuration of the fluid flow circuit 10 and the attached modules.

Various additional components may be incorporated into the fluid flow circuit. For example, a return line filter may be associated with a line leading to a fluid recipient, filters may be positioned upstream of one or more of the fluid containers to remove a substance (e.g., leukocytes) from a separated component (e.g., red blood cells or platelets) flowing into a reservoir. Filters may also be present on the lines of one or more containers used for addition of materials, such as containers 40, 46, 48, 64, 66, 68, and 70 in order to filter or sterilize materials as they enter the fluid circuit. Additionally, a cell counting component may also be added. A spill detection module or device may also be included.

Before beginning a blood processing procedure, if there are any fluid containers that are not integrally formed with the fluid flow circuit 10, they may be connected to the fluid flow circuit 10 (e.g., by piercing a septum of a tube of the fluid flow circuit 10 or via a luer connector), with the fluid flow circuit 10 then being mounted to the blood separation device 20. Additionally, any source containers may be filled with the appropriate fluid, such as buffer or solution. An integrity check of the fluid flow circuit 10 may be executed by the controller 16 to ensure that the various components are properly connected and functioning.

To begin a blood processing procedure, an operator may select (e.g., using the user interface screen 14 of Fig. 3C) the procedure from among the variety of procedures that the device 20 is capable of performing. The operator may enter a variety of information requested by the system controller 16 that allows the controller to better carry out the procedure. The controller can be provided with the desired cell modification processes, the solutions being use, type of blood cells being modified, total blood volume needed for the process or of the blood source, a WBC pre-count, WBC subset pre-count or the initial WBC concentration of the blood of the blood source, and a WBC post-count or a target platelet concentration to be achieved for the blood of the blood source by the end of the procedure. The total amount of blood to be processed may also be provided to the system controller. Additionally, various patient measurements such as height, weight, etc. may also be added.

When the system controller has received all of the necessary input, performed the necessary preliminary calculations and status checks (e.g., to confirm that the flow circuit 10 is properly installed and that the various components of the system 21 are functioning properly), the blood source is connected to the fluid flow circuit 10 (e.g., by phlebotomizing a patient or attaching a whole blood container), and a blood processing procedure may begin. The blood source may include a patient or a container of blood or other suspension of cells. The blood or other cellular starting material may be whole blood, blood components obtained by, for example, apheresis, or other nucleated cell suspensions.

Blood is introduced into the system by either an active or passive user-adjacent portion of the fluid flow circuit 10 or by connection of a blood or blood component container. The blood flows from the donor into the main line L1 and reservoir 40. In an initial stage, which is referred to herein as a "blood prime" stage and shown in Fig. 9, selected components of the fluid flow circuit 10 are primed using blood 41 from a blood source, particularly being stored in reservoir 40. It is also within the scope of the present disclosure for the fluid flow circuit 10 to be primed using a different priming fluid, such as saline.

During the blood prime stage, whole blood is drawn into the fluid flow circuit 10 from the reservoir 40 via line L2. The blood travels through L1 to the patient-adjacent portion of the fluid flow circuit 10. Although not shown in Fig. 9, the priming process may be done to other components of the fluid flow circuit, such as parts of the processing portion 25 of the fluid flow circuit 10.

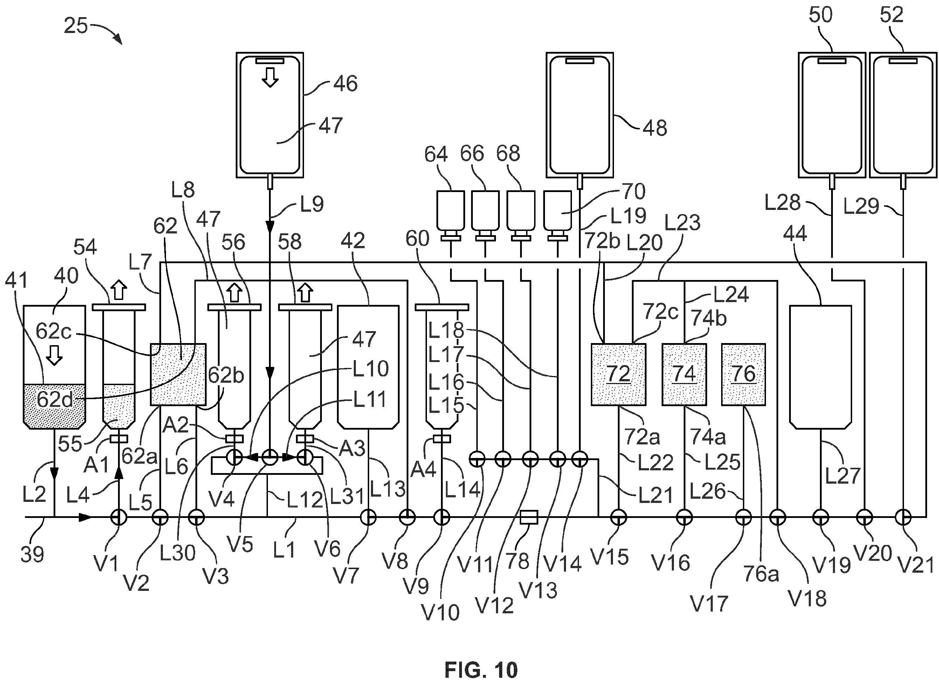

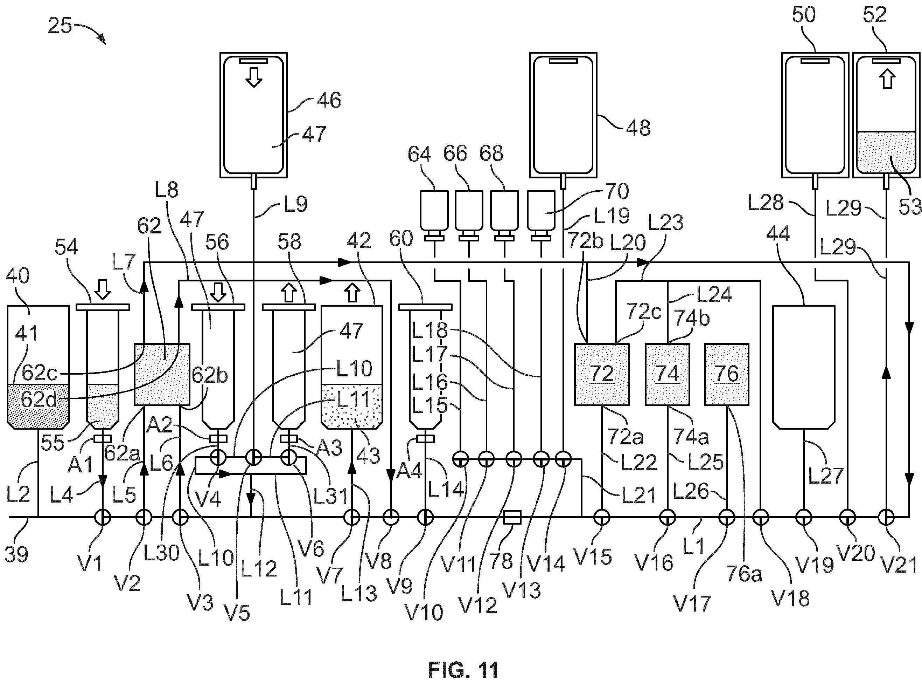

A separation stage of the procedure may then be initiated by the controller 16. In a first step of the stage, shown in Fig. 10, blood 41 from reservoir 40 is loaded into pump 54 and buffer 47 from container 46 is loaded into pumps 56 and 58. Blood or blood components 41 from reservoir 40 is/are pulled or loaded into pump 54 by passing through lines L2 and L1, valve V1, and line L4. The blood or blood components in the pump 54 are identified at 55 in Figs. 10 and 11. The buffer 47 is passed from container 46 through line L9 and valve V5 to either line L10, through valve V4 and line L30 to pump 56 or to line L11, through valve V6 and line L31 to pump 58. Buffer 47 may optionally be also directed to the pump 54. In a second step of the separation stage, shown in Fig. 11, the pumps 56 and 58 are pressurized to operating pressure to convey buffer 47 into the separator module 62. Pumps 56 and 58 may be sequenced in order to provide continuous flow. Buffer 47 is pushed from either pump 56 or pump 58 through line L12, valve V3, and line L6 to the separation module 62 at inlet 62b. Pump 54 also pushes blood or blood components 55 through line L4, valve V1, valve V2, and line L5 into separation module 62 at inlet 62a. The cells are separated based on size. Larger cells/particles 43, such as white blood cells, are separated and directed into reservoir 42 through outlet 62d, by line L8 through valves V8 and V7 to line L13. Smaller cells/particles 53, such as red blood cells and platelets are directed to the waste container 52 by traveling out of outlet 62c to line L7, line L1, valve V21, and line L29 to waste container 52. The separation module may perform differently depending on the desired separation and particular cell to be modified.

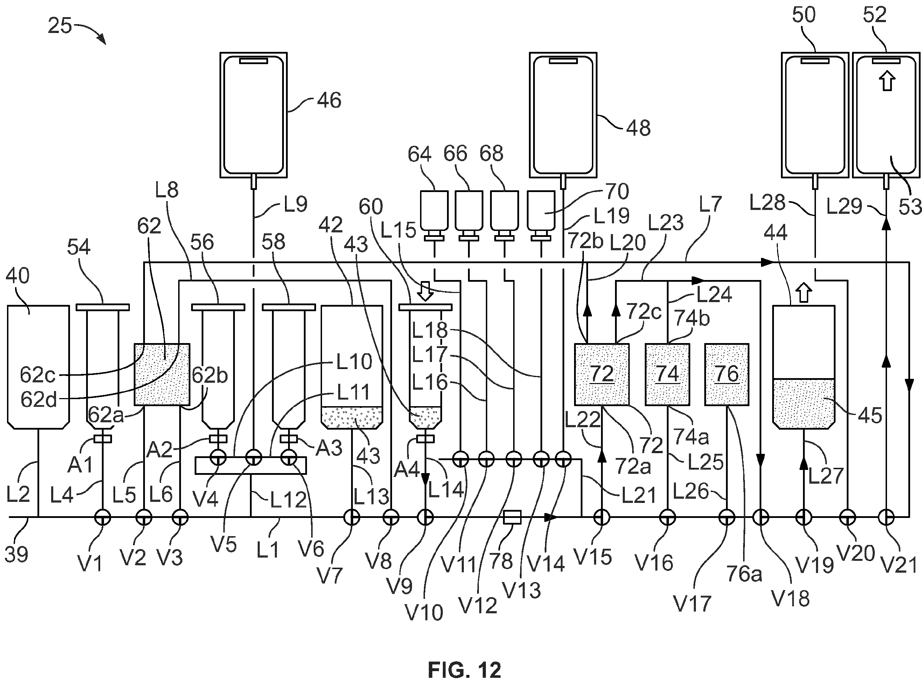

A cell concentration procedure may then be performed. Large cell material 43 is loaded or pulled into pump 60 from reservoir 42 by passing through line L13, valves V7, V8, and V9 to line L14. Then, as shown in Fig. 12, pump 60 is pressurized to operating pressure and the large cell material 43 is pushed through line L14 and valve V9 to line L1 and then through valve V15 and line L22 to the inlet 72a of the concentrator module 72. The concentrator module produces concentrated cells 45 and supernatant 59. Supernatant 59 is directed towards the waste container 52 by passing through outlet 72b to line L20 to line L7, through valve V21 and to line L29. Concentrated cells 45 are directed towards reservoir 44 by passing out of outlet 72c to line L23, through valve V18, line L1, and valve V19 to line L27. Concentrated cells may be subsequently diluted, then optionally reconcentrated by drawing again into pump 60 from reservoir 44 and repeating the cell concentration procedure until cells are at a target concentration. Cell concentration may be sensed during concentration or transfer states.

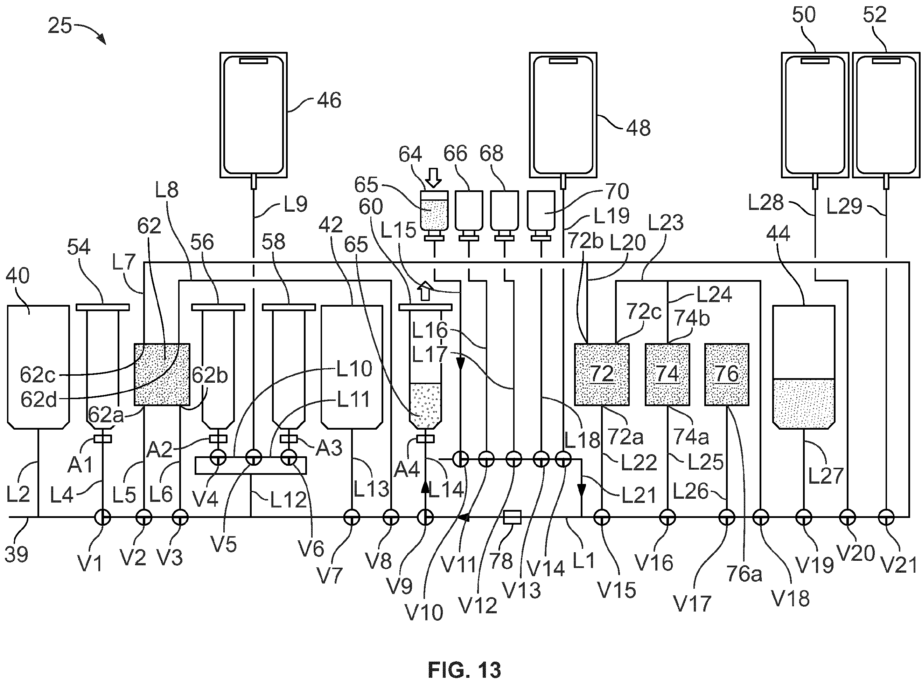

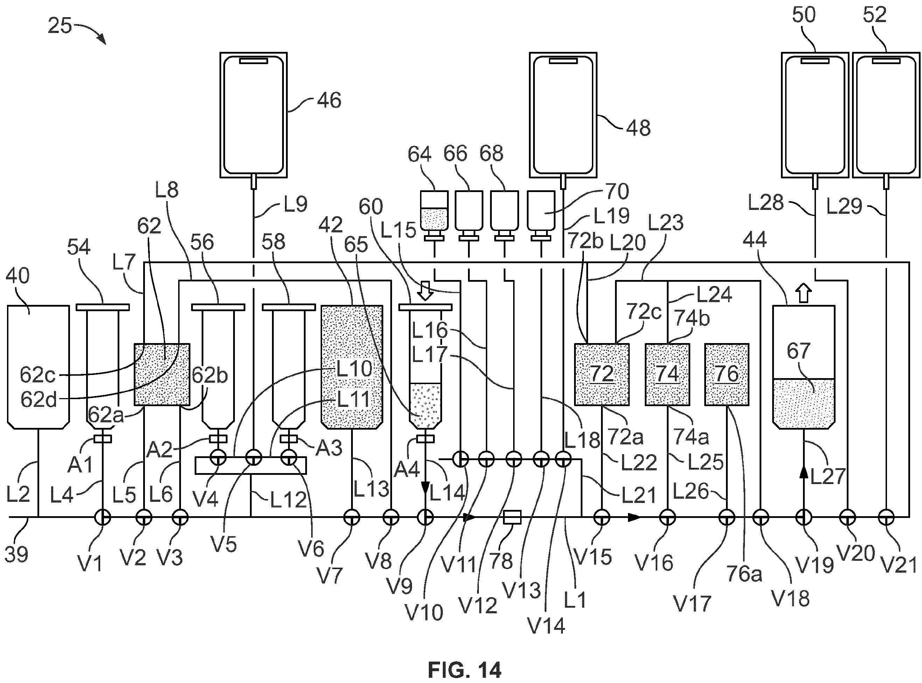

A cell formulation stage or procedure may also be performed. This may be done to concentrated cells 45 (as shown in Figs. 13 and 14) or on the separated cell material (such as large cell material 43). Concentrated cells 45 or large cell material 43 may be treated with at least one solution, buffer, or combinations of solution(s) and buffer(s) in reservoirs 42 and 44. In an exemplary method, a first step, shown in Fig. 13, includes a measured volume of solution 65 being drawn into pump 60. The solution 65 from container 64 travels through line L15, valves V10, V11, V12, V13, and V14 to line L21 and L1 to valve V9 and line L14. In a second step of the cell formulation stage of the exemplary method, shown in Fig. 14, the solution 65 in pump 60 is pumped toward reservoir 44, which contains the concentrated cells 45. Specifically, the solution travels through line L14 and valve V9 to line L1, through valves V15, V16, V17, V18, and V19 to line L27. The concentrated cells 45 combine with the solution 65 and may be incubated into suspension 67. During hold or incubation steps, the suspension 67 may be drawn back and forth between reservoir 44 and pump 60 to prevent settling. In an alternative second step of the cell formulation stage (when solution 65 is to be added to large cell material 43), solution 65 may be instead pumped toward reservoir 42, where the large cell material 43 is held after the separation stage (Fig. 11). Specifically, the solution travels through line L14 and valve V9 to line L1, through valves V8 and V7 to line L13. The larger cells 43 combine with the solution 65 and may be incubated in reservoir 42. During the incubation, the suspension may be drawn back and forth between reservoir 42 and pump 60 to prevent settling.

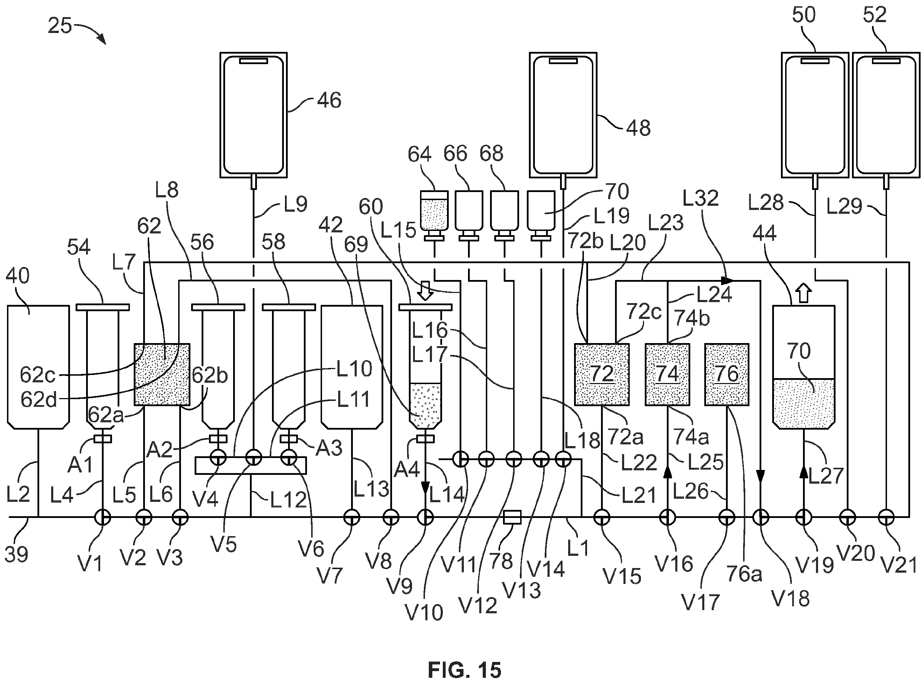

When cell modification involves the introduction of a therapeutic payload, a delivery stage procedure may also be performed, as shown in Fig. 15. Cells 69 which have been formulated with a modification solution can be directed from pump 60 to the delivery module 74 in which payload is administered to the cells. Similar to the process shown in Figs.13 and 14, cells 69 may be formed by first pulling a modification solution into pump 60 and pushing the solution into one of reservoir 42 or 44, which include cellular blood components. These cells 69 can be formed from the large cell material 43 or cells which have already been modified, such as by the cell concentrating or formulating stage. The cells 69 may then be pulled into pump 60. As shown in Fig. 15, the formulated cells pass from pump 60 through line L14 and valve V9 to line L1, through valves V15 and V16 to line L25 and into delivery module 74 through an inlet 74a. This module 74 may leverage electroporation, mechanoporation, or other flow-through transfection methods to introduce the payload. The modified cells 70 exit the module 74 at outlet 74b to line L32 and line L23 through valves V18 and V19 to line L27.

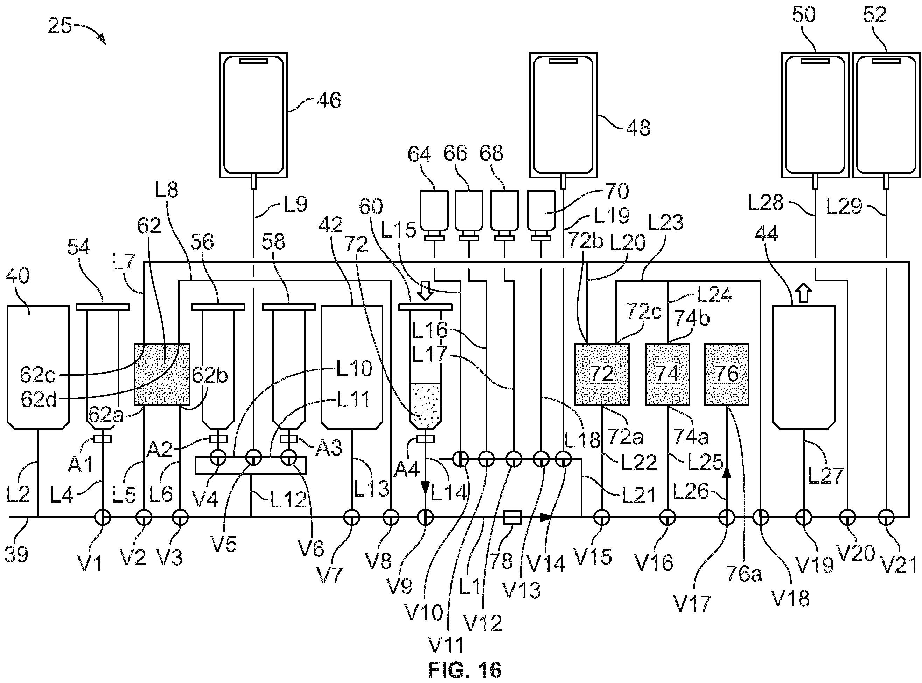

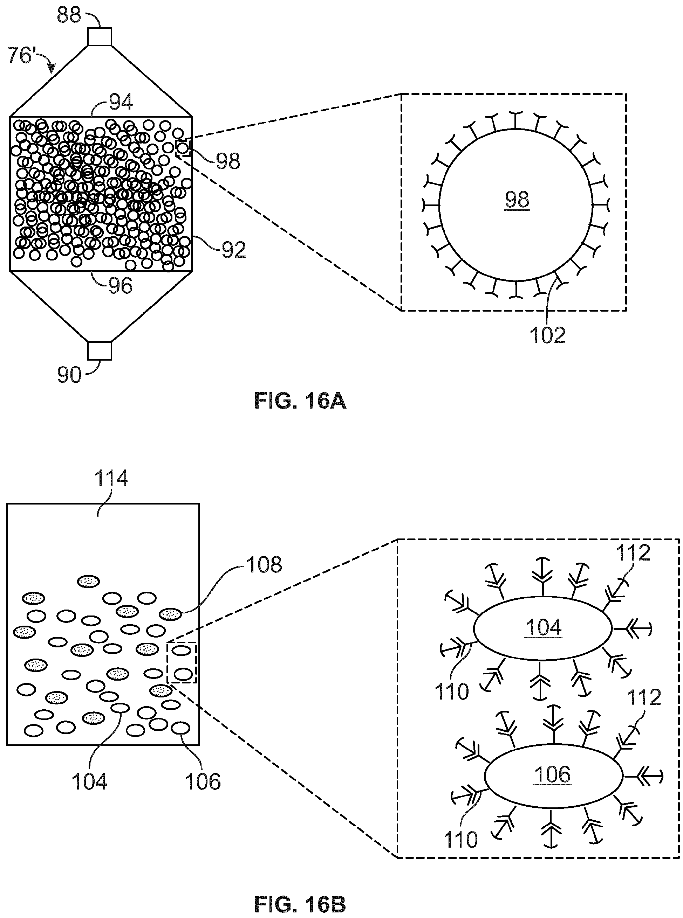

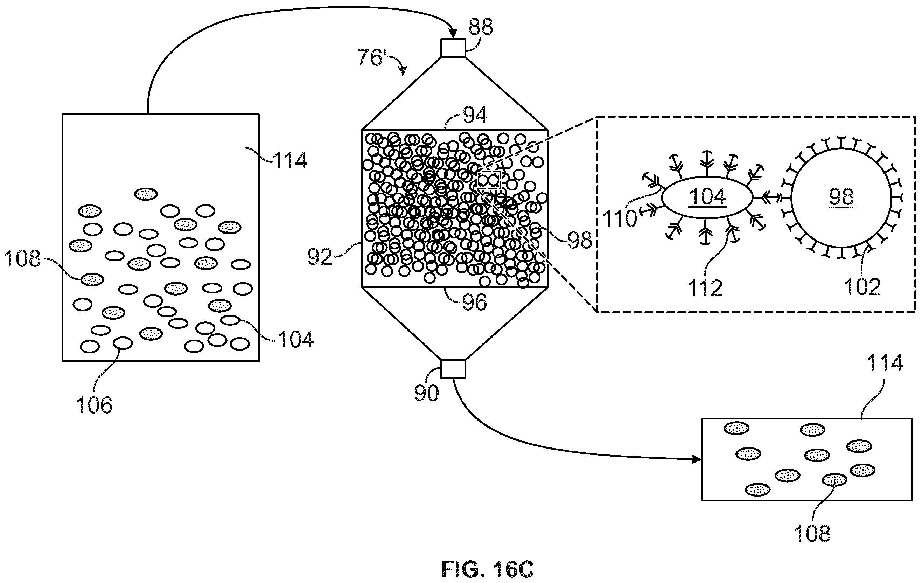

A delivery stage procedure may also be accomplished without passing through the delivery module 74. Instead, a delivery solution is simply added in the above-described cell formulation stage (to large cell material 43). For instance, lipid nanoparticles may incubate with the cells in order for them to introduce their payload. Optionally, a cell selection stage procedure may also occur or be performed. Cells 72 which have been formulated with antibodies, beads, or other solutions that can identify cells based off surface markers or phenotype can be passed into a selection chamber 76. Similar to the process shown in Figs. 13 and 14, cells 72 may be formed first by pulling the cell identifying solution into pump 60 and pushing the solution into one of reservoirs 42 or 44, which include blood component cells. These cells 72 can be formed from the large cell material 43 or cells which have already been modified, such as by the cell concentrating stage, formulating stage, or delivery stage. The cells 72 may then be pulled into pump 60. As shown in Fig. 16, the cells 72 pass from pump 60 through line L14 and valve V9 to line L1, through valves V15, V16, and V17 to L26 and into the cell selection module 76 through an inlet 76a. Cells can be positively or negatively isolated and the target cell fractions can be directed towards an appropriate reservoir or output container.

Fig. 16A illustrates an exemplary cell selection chamber 76' that may be used during the cell selection step of Fig. 16, as the cell selection module 76. While Fig. 16 illustrates a cell selection module 76 including a single entry point 76a into the cell selection module 76, it will be seen that the cell selection chamber 76' of Fig. 16A includes an inlet 88 and an outlet 90. In such an embodiment, the cell selection chamber 76' may be incorporated into the fluid flow circuit of Fig. 16 with one of the inlet 88 and the outlet 90 connected to line L26 and the other one of the inlet 88 and the outlet 90 connected to line L23. It will be seen that the cell selection chamber 76' is symmetrical, such that fluid may flow through it from the inlet 88 to the outlet 90 or from the outlet 90 to the inlet 88 without affecting its operation. Thus, during the cell selection step, fluid may flow from line L26 through the cell selection chamber 76' and then into line L23 (in which case the lower port 90 that is identified as the "outlet" will instead serve as a fluid inlet and the upper port 88 that is identified as the "inlet" will instead serve as a fluid outlet) or may instead flow from line L23 through the cell selection chamber 76' and then into line L26 (with the inlet 88 acting as a fluid inlet and the outlet 90 acting as a fluid outlet).

The cell selection chamber 76' includes a reservoir 92 positioned between the inlet 88 and the outlet 90, with the reservoir 92 defining a conduit or vessel allowing for fluid flow from the inlet 88 to the outlet 90 (or from the outlet 90 to the inlet 88). An upstream mesh or membrane 94 is positioned between the inlet 88 and the reservoir 92, while a downstream mesh or membrane 96 is positioned between the outlet 90 and the reservoir 92. Each of the porous membranes 94 and 96 extends to the inner perimeter of the cell selection chamber 76', such that any fluid flowing into the reservoir 92 must pass through one of the membranes 94, 96 (via the plurality of pores defined in that membrane) and any fluid flowing out of the reservoir 92 must pass through the other membrane 94, 96 (via the plurality of pores defined in that membrane). The upstream membrane 94 may be differently configured from the downstream membrane 96, though it may be advantageous for the membranes 94 and 96 to be substantially identical to enhance the symmetry of the cell selection chamber 76' and promote fluid flow through the cell selection chamber 76' in either direction.

The reservoir 92 contains a plurality of microbubbles 98. The membranes 94 and 96 serve to retain the microbubbles 98 within the reservoir 92, such that the plurality of pores defined in the membranes 94 and 96 will have diameters that are less than the diameter of each of the microbubbles 98. In one exemplary embodiment, each microbubble 98 has a diameter that is greater than 50 microns, while each membrane pore has a diameter of less than 50 microns, thereby preventing the microbubbles 98 from exiting the reservoir 92 through a membrane pore. In other embodiments, the microbubbles 98 and membrane pores may have other diameters (e.g., microbubbles 98 having a diameter in the range of approximately 10 - 200 microns or, more preferably, in a range of approximately 25 - 75 microns or, more preferably, in a range of approximately 40 - 60 microns, with the membrane pores having a smaller diameter than the microbubbles 98 in each case), which may include all of the microbubbles 98 retained within the reservoir 92 having the same diameter or at least one of the microbubbles 98 retained within the reservoir 92 having a diameter that is different from the diameter of another microbubble 98 retained within the reservoir 92. One constraint on the configuration of the membrane pores is the nature of the cells to be conveyed into the cell selection chamber 76', as the membrane pores should have diameters that are sufficiently large to allow for the cells to pass through the membranes 94 and 96, rather than the membranes 94 and 96 preventing flow of the cells through the cell selection chamber 76'. In one exemplary embodiment, the membrane pores have a diameter of at least 10 microns and in the range of approximately 25 - 98% of the diameter of the smallest associated microbubbles 98 (e.g., 25 - 49 micron pores and 50 micron microbubbles 98). In another exemplary embodiment, the membrane pores have a diameter of at least 10 microns and in the range of approximately 15 - 90% of the diameter of the smallest associated microbubbles (e.g., 30 - 45 micron pores and 50 micron microbubbles 98). In yet another exemplary embodiment, the membrane pores have a diameter of at least 10 microns and in the range of approximately 50 - 80% of the diameter of the smallest associated microbubbles 98 (e.g., 35 - 40 micron pores and 50 micron microbubbles 98).

The microbubbles 98 may be formed according to any suitable approach and formed of any suitable material (e.g., silica) without departing from the scope of the present disclosure, which may include the microbubbles 98 being of the type manufactured by Akadeum Life Sciences of Ann Arbor, Michigan. Each of the microbubbles 98 is at least partially coated (and, more preferably, entirely coated) with streptavidin. The streptavidin coating 102 may be applied to the microbubbles 98 according to any suitable approach without departing from the scope of the present disclosure.

Fig. 16B illustrates a heterogeneous population of cells to be conveyed into the cell selection chamber 76' during the cell selection step. Fig. 16B illustrates the heterogeneous population of cells as including three different types of cells 104, 106, and 108, though it should be understood that a heterogeneous population of cells may include any number of types of cells (except for a single type of cells). The particular types of cells making up the heterogeneous population of cells may vary without departing from the scope of the present disclosure, provided that at least one of the types of cells (which are identified in Fig. 16B at 104 and 106 and may be referred to herein as "target cells") has surface antigens 110 that will bind to regions 112 of a biotinylated antibody additive (identified in Fig. 16B at 114) with which the cells are mixed and at least one of the types of cells (identified in Fig. 16B at 108) does not have such a compatible surface antigen. In one embodiment, the heterogeneous population of cells includes CD3+ cells, with the biotinylated antibody additive binding to all of the cells (which may be referred to as "target cells") except for the CD3+ cells (which may be referred to as "unbound cells").

As described above, the cell selection step may include a cell identifying solution (which may be the biotinylated antibody additive 114 of Fig. 16B) being pulled into a pump and then conveyed from the pump into a reservoir container holding a population of cells (which may be the heterogeneous population of cells of Fig. 16B). The mixture of cells and solution may then be conveyed from the reservoir container into one of the pumps before the mixture is conveyed into the cell selection module. Consistent with the above description of the cell selection step, it should be understood that Fig. 16B may represent the mixture of cells and biotinylated antibody additive 114 being positioned within a reservoir container or a pump (prior to being conveyed into the cell selection chamber 76').

With the regions 112 of the biotinylated antibody additive 114 bound to the target cells 104 and 106 and not to other cells 108 (the "unbound cells"), the mixture of the biotinylated antibody additive 114 and the heterogeneous population of cells is conveyed into the cell selection chamber 76', as shown in Fig. 16C. The mixture passes into the cell selection chamber 76' via whichever port is selected as the inlet (which is port 88 in Fig. 16C), passes through the pores of the adjacent membrane (which is the upstream membrane 94 in Fig. 16C), and then enters the reservoir 92 of the cell selection chamber 76', where it is exposed to the coated microbubbles 98. Within the reservoir 92 of the cell selection chamber 76', the target cells 104 and 106 carrying the biotinylated antibody 112 become bound to the streptavidin coating 102 of the microbubbles 98, thus entrapping the target cells 104 and 106 within the reservoir 92 of the cell selection chamber 76', with the microbubbles 98.

The unbound cells 108 (i.e., the ones not carrying the biotinylated antibody 112) will pass through the reservoir 92 of the cell selection chamber 76' without becoming bound to the microbubbles 98, exiting the reservoir 92 via the other membrane (which is the downstream membrane 96 in Fig. 16C) and then exiting the cell selection chamber 76' via the port that is selected as the outlet (which is port 90 in Fig. 16C). The unbound cells 108 exiting the cell selection chamber 76' may be conveyed into a suitable container, such as one of the reservoir containers, for subsequent processing (which may include, for example, the unbound cells later being modified and administered to a recipient).

Although possible cell modification stages have been described, it is not required that each stage be performed on the collected blood cells in any given blood processing procedure, but may depend on the resulting cell make-up requirements. The cells may be modified in at least one stage and can be modified in all stages, or a combination of some of the stages. The stages do not have to be performed in a specified order and can each be done multiple times. Most importantly, the cells are separated and modified in the same procedure and system.

Once the cells are modified in at least one of the described cell modifying modules, these cells may be directed to the dose/sample container 50 (Fig. 8) or optionally returned to the donor/patient through the fluid flow circuit. If directed to container 50, the container may be removed from the circuit. The modified cells may pass back through to the donor/patient by directing the cells back through line L1 of the processing portion 25 of the fluid flow circuit 10 to the patient-adjacent portion 22 of the fluid flow circuit. Various pumps and valves may be utilized to direct the fluid back to the patient. The patient-adjacent portion 22 of the fluid flow circuit may initiate and direct the fluid flow back to the patient.

There are a few factors that may be used to determine whether the cells are collected in a dose or reinfused back to the patient. These include guidelines imposed by regulators (like the FDA), the duration of processing that is required, whether patient connection for the duration is acceptable (e.g., if the processing takes hours, perhaps the patient need not be connected during the duration of processing), the need for extensive release testing prior to reinfusion, or if the cells require additional handling (such as culture/expansion, offline dosing, etc.).

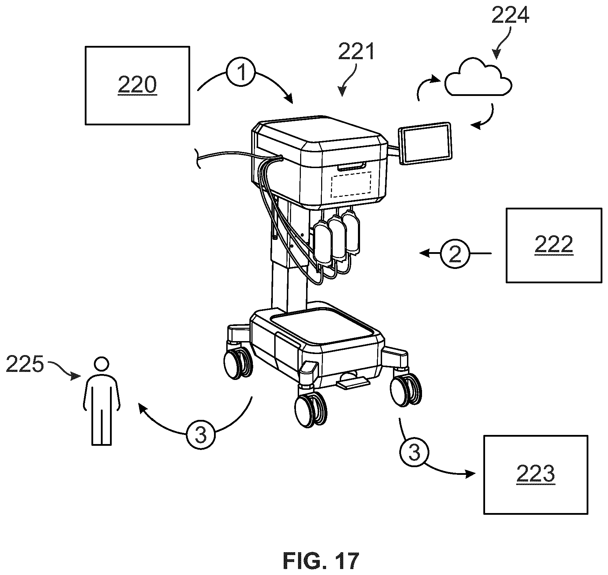

A more particular embodiment of the systems and methods described above is depicted in Figures 17-22 and will now be described. It will be understood that such systems and methods may utilize many of the aspects and features described previously to provide a single, modular system configured for point-of-care (e.g., bedside or chairside) use. For example, device 221 shown in Fig. 17 may be the mobile modular device 20 and system 21 of Figs. 2, 3B and 3C. Fig. 17 shows biological cell processing system 221 including the device 20 and the fluid flow circuit 10 of the current disclosure and a possible procedure indicated at steps 1-3. The blood processing system receives blood such as whole blood, blood components (which may typically be obtained by apheresis), or other nucleated cell suspensions 220 from a source at step 1, wherein the source may be a patient or a container of previously collected blood or blood component(s). The system uses protocols or procedures from internal or external data sources 224 and performs various processes in the system. The system can also share generated data (at this step or a further step) with external sources 224. Data may be instrument statuses, error logs, procedure data/records, or live sensor data. At step 2, additional fluids or agents, such as additives or solutions 222 are added to the system, through at least one module. There are two alternative step 3's in which the final product can be returned to a patient 225 or collected in a bag or other output receiver 223. This entire process may take place in less than a day, less than 12 hours, or even under 6 hours.

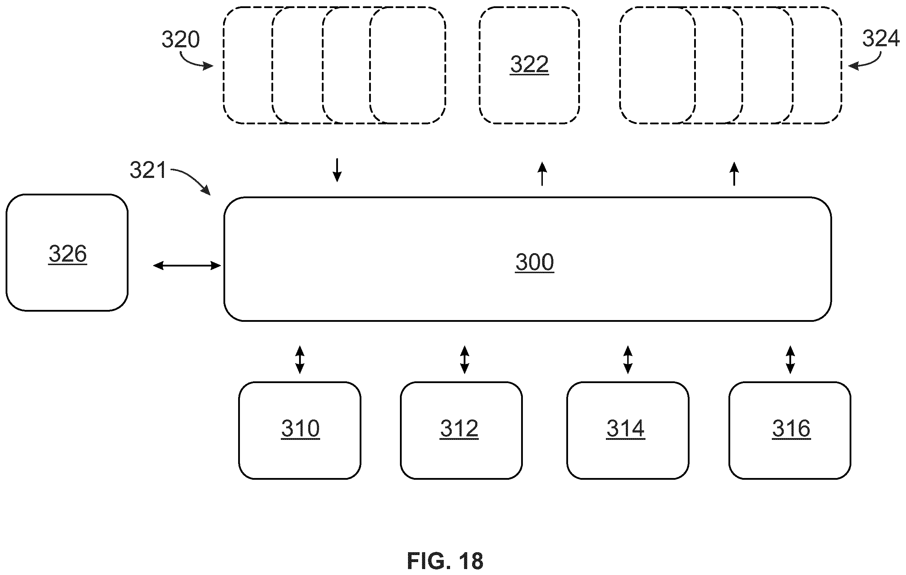

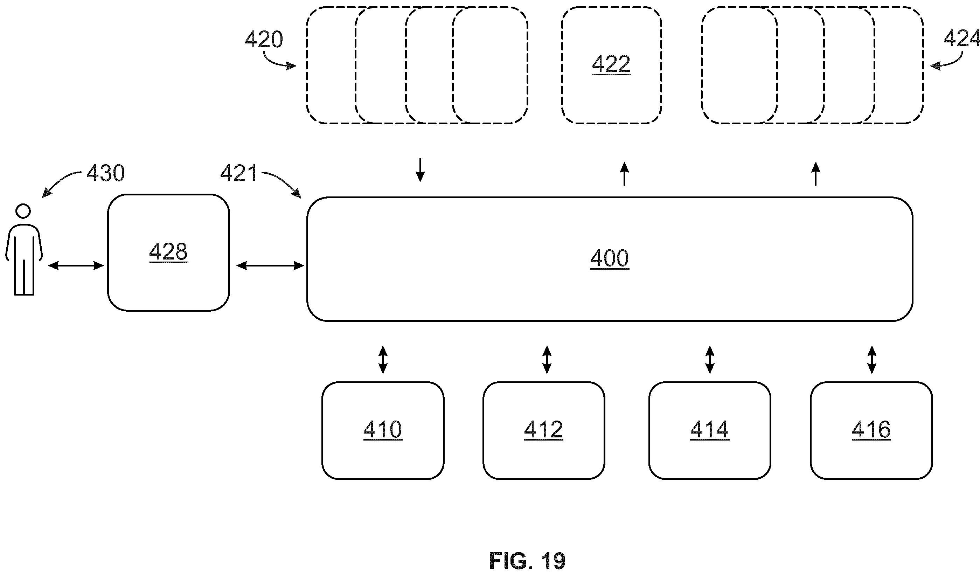

Figs. 18 and 19 show two block diagrams of the blood processing system, with the difference being the type of blood source utilized. In particular, system 321 includes as its source a bag or container with previously collected blood, blood components obtained by apheresis, or other nucleated cell suspensions 326 (obtained, for example, by apheresis), while and system 421 utilizes a donor 430 and includes a donor management module 428. The donor management module can be either of the passive or active user-adjacent portions of the fluid flow circuit discussed above and shown in Figs. 6 and 7. The systems 321 and 421 each include a fluid handling and control system 300, 400, as a central and holding component for the other modules and inputs/outputs. A plurality of different modules may be added or removed from the system (such that only one, two, or three modules may also be present instead of all four). The modules may include a sorter 310, 410 (e.g., a microfluidic sorter or a cell selection chamber of the type described above), a microfluidic concentrator 312, 412, and optionally a cell selection module 314, 414, and a "cargo delivery" module 316, 416. Inputs to the system can include processing buffers and solutions 320, 420. Outputs of the system can include waste material 322, 422 and samples or outputs 324, 424.

While the systems 221, 321, and 421 may utilize microfluidic portions, the systems may instead be configured to allow for processing of larger amounts of whole blood or blood components obtained from whole blood. The flow rates through the system may be from 5 mL/min to 70mL/min, for example. The systems 221, 321, and 421 as a whole can process up to 500 mL of whole blood, with the withdrawal or draining comprising up to an hour from the blood source.

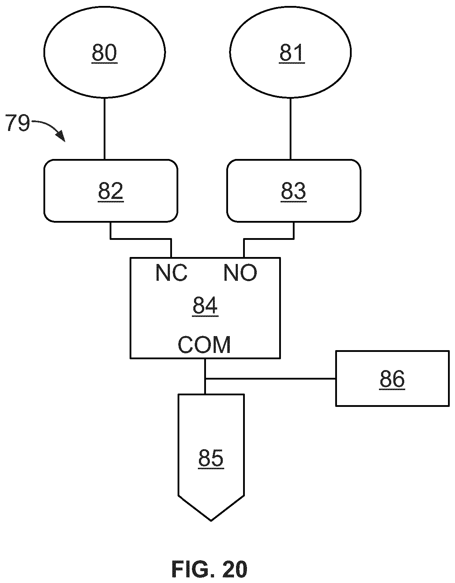

Systems 221, 321, and 421 may utilize pneumatic syringe pumps as described above for delivering starting cellular material, buffers or other agents and additives to the sorter and concentrator. The system may further include a pressure control system 79, shown in Fig. 20. The pressure control system 79 of syringe pump 85 includes a pressure tank 80 and a vacuum tank 81, with associated pressure regulator 82 and vacuum regulator 83. A three-way valve 84 includes connections that are normally open (NO), normally closed (NC), and for common (COM). The system 79 further includes a two-way normally open valve 86 which can function as a vent. These tanks 80 and 81 are maintained at 90-110 psi and <-10 psi, respectively, in one embodiment. Each syringe has a syringe controller which has a regulator to set syringe pressure.

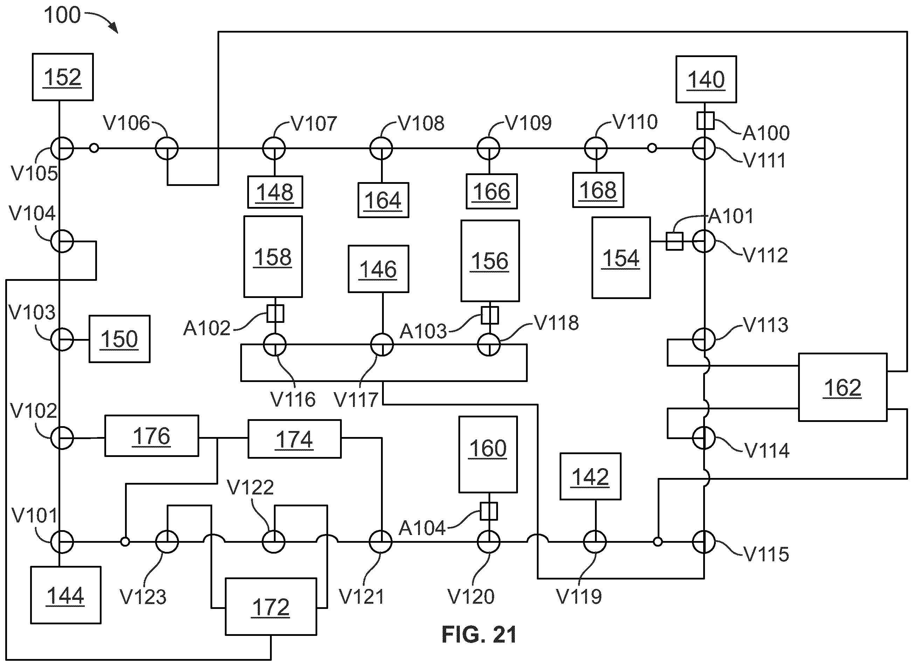

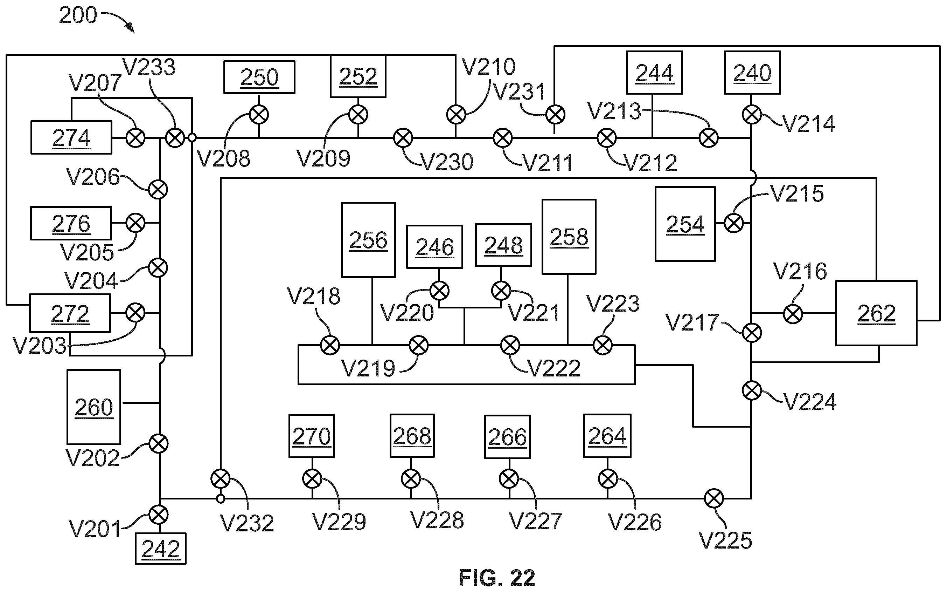

The valve system of exemplary embodiments of the blood processing system 221, 321, and 421 can include stopcock valves or solenoid valves. Fluid flow circuits 100 and 200 in Figs. 21 and 22 show fluid flow paths with stopcock valves (Fig. 21) and solenoid valves (Fig. 22), respectively.

More particularly, Fig. 21 shows a fluid flow circuit 100 of a blood processing system of the current embodiment. Valves V101-V123 can be any known type of stopcock valve. The fluid flow circuit 100 connects and established fluid communication between and among component parts, similar to those described above including: a first buffer container 146, a second buffer container 148, reservoirs 140, 142, and 144, a sample/dose container 150, a waste container 152, pumps 154, 156, 158, and 160, and solution or liquid containers 164, 166, and 168. The fluid flow circuit may also include air detectors A100-A104. The fluid flow circuit 100 further includes a sorter module 162, a cell concentrator module 172, and optionally a cargo delivery module 174, and a cell selection module 176.

Fig. 22 shows a fluid flow circuit 200 of a blood processing system of the current embodiment. Valves V201-V233 can be any known type of solenoid valve. The valves may be part of a solenoid-driven cassette, of the type shown in U.S. Patent Application Publication No. 2017/0290972, which is hereby incorporated herein by reference. The fluid flow circuit 200 connects and established fluid communication between and among component parts, similar to those described above including: a first buffer container 246, a second buffer container 248, reservoirs 240, 242, and 244, a sample/dose container 250, a waste container 252, pumps 254, 256, 258, and 260, and solution or liquid containers 264, 266, and 268. The fluid flow circuit 200 may also include air detectors, although not specifically illustrated. The fluid flow circuit 200 further includes a sorter module 262, a cell concentrator module 272, and optionally a cargo delivery module 274, and a cell selection module 276.

The sorter module 162, 262 may have properties that are common with the above-described separation module, such as shown in Fig. 8 in the schematic of the fluid flow circuit. This sorter module 162, 262 may be a substantially or completely passive separation operation, without reliance on moving parts or complex systems, such as that in a centrifugal separation or spinning membrane separation. In an embodiment, the sorter module may have a microfluidic configuration, being provided as a chip or cartridge. If provided as a chip, the chip may have multiple layers including an interface layer, which enables fluidic connections to other components, such as tubing. The chip may also have a lid layer for sealing the lower fluidic layer. The fluidic layer includes the microfluidic channels and carries out the cell sorting and concentration. The microfluidic chip or cartridge may operate to sort the cells based on inertia. If whole blood is introduced to a sorter module having a microfluidic configuration, dilution is required, whereas apheresis product does not require the same dilution. Microfluidic chips or cartridges utilized in the microfluidic sorter module can be of the type described in U.S. Patent No. 10,150,116, which is hereby incorporated herein by reference.

The cell concentrator module 172, 272 may include the properties described above in relation to the concentrating module, such as shown in Fig. 8 in the schematic of the fluid flow circuit. The cell concentrator module 172, 272 may include a number of concentrator channels. In one embodiment, the concentrator includes at least 200 channels, and in another embodiment includes at least 250 concentrator channels. The concentrator may operate passively, without any moving parts or complex control systems. The concentrator module may operate at greater than 100 mL/min. The cell concentrator may operate to concentrate at least one cellular component by 10x. In one example, the sorter module separates nucleated cells from anucleated cells. These nucleated cells can then be concentrated in the cell concentrator module. The cell concentrator module may include the cell concentrator technology of the type described in U.S. Patent No. 10,150,116. The microfluidic sorter module and cell concentrator module may be connected and added and removed from the system as one piece or they may each be separate pieces.

The fluid flow circuit may include a cargo delivery module 174, 274 or feature which utilizes microfluidic mechanoporation in order to accomplish intracellular cargo (for example, gene) delivery. Cells and target material in suspension undergo high speed cell deformation which causes a temporary disruption of the cell membrane and allows the target material to enter the cell. Afterward, the membrane reseals. The cargo delivery module 174, 274 may include delivery materials such as mRNA, siRNA, saRNA, polymers, proteins and peptides, antibodies, viruses, labelling molecules, small molecules, and CRISPR RNPs. The validated cell types may include peripheral blood mononuclear cells, T-cells, B-cells, monocytes, natural killer (NK) cells, hematopoietic stem cells, induced pluripotent stem cells, and red blood cells.

The fluid flow circuit may include a cell selection module 176, 276 which may be configured according to the above description of a cell selection chamber 76' or may be differently configured, such as the cell selection module being column-based and have immunophenotypic cell selection. In such an embodiment, the cell selection module 176, 276 may include a non-magnetic affinity-chromatography based cell selection column that operates to isolate a target cell (such as CD3+) from a population including non-target cells. The process uses a polymer matrix with a ligand and ligand binding partner which binds the target antigen (target cell, CD3+). A competing molecule is then added to release the target cell. The resulting target cells may require a buffer rinse to be label-free. The cell selection technology utilized can be that described in Next Generation Automated Traceless Cell Chromatography Platform for GMP-compliant Cell Isolation and Activation. Scientific Reports (2022) 12:6572.

Any components not specifically described in relation to the blood processing systems 221, 321, and 421 are the same or similar to those described above in relation to the blood processing system 21. The blood processing systems 221, 321, and 421 include at least a controller and a fluid flow circuit 100, 200. The fluid flow circuit includes at least a sorter module, a cell concentrator module, at least one pump, at least one fluid reservoir for holding fluids during a blood processing procedure, a valve system, at least one fluid source container, a blood source access device, and a plurality of conduits fluidly connecting the components of the fluid flow circuit. The fluid flow circuit may optionally include at least one cell modification module, such as a cargo delivery module and (preferably) a cell selection module. The blood processing system may include further components such as sensors, air detectors, additional pumps, and weigh scales, as described above.

### Example

The following collection and processing workflow could be accommodated using combinations of the states and procedures previously described. Using a fluid flow circuit, the system collects a target volume of anticoagulated whole blood from a patient based on target cell peripheral blood pre-counts into reservoir 40. The anticoagulated whole blood is separated in the separation module and white blood cells are isolated in reservoir 42. The white blood cells are then passed through the concentration module 72 and concentrated 25x, which may include multiple passes through the concentration module. A biotinylated antibody additive is introduced into the system and the white blood cells are incubated in reservoir 44 with the biotinylated antibody additive, which is used to label all of the cells except for CD3+ cells. Afterward, the mixture is conveyed through the selection module, where the labeled cells become bound to the streptavidin-coated microbubbles and are retained in the module. The unlabeled, unbound CD3+ cells are removed from the selection module into a reservoir such as reservoir 42 or 44. The CD3+ cells are then passed into the concentration module 72, where they are 10x concentrated. A modification solution (e.g., mRNA, CRISPR-Cas9, Transposon/Transposase) is introduced into the system and into the holding reservoir with the concentrated CD3+ cells. The mixture of solution and CD3+ cells is then transferred through the delivery module 74, with the CD3+ cells being directed into another reservoir as modified cells. These modified cells then can be washed in the separation module 62 with buffer. The modified cells are then passed through the cell concentration module 72 and concentrated to a target reinfusion target. These concentrated cells are then transferred to a dose container or reinfused into the patient.

Thus, an improved method and system have been disclosed for the processing of blood components. Advantageously, the modular fluid flow circuit described herein includes cell separation, cell selection, and cell modifying components and can produce separated and modified cells in a single modular system, in one procedure. The description provided above is intended for illustrative purposes only and is not intended to limit the scope of the invention to any specific method, system, or apparatus, or device described herein except as may be explicitly delineated above.

### Possible Modified Cells And Uses For Such Cells

In one embodiment, the modified cells produced by the blood processing system include Chimeric Antigen Receptor T-Cells (CAR-T) cells. These CAR-T cells may be used in several applications and therapeutic treatments of various diseases, ailments, or conditions. For example, the CAR-T cells may be used to treat solid tumors such as anal/rectal, epithelial, ovarian, breast, fallopian tube, endometrial, pancreatic, colorectal, lung, and/or gastrointestinal tumors. The CAR-T cells may also be useful in therapeutically treating melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, and rheumatoid arthritis. The CAR-T cells may also be useful for renal transplantation patients. Accordingly, the disclosure provides a modified CAR-T cell produced by the blood processing system for use in the treatment of a disease, ailment, or condition selected from the group consisting of solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation.

In producing the CAR-T cells, different modification strategies may be used in the blood processing system, with the various methods being practiced in the preparation of CAR-T cells for any one of the preceding applications or therapeutic treatments. Vectors such as mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs may be utilized in the blood processing system (specifically the cargo delivery module) to create the CAR-T cells. Accordingly, the disclosure provides in certain embodiments modified CAR-T cells produced by use of the aforementioned vectors for use in the treatment of a disease, ailment, or condition as shown in Tables 1 and 2:

**Table 1**

| **Embodiment #** | **Disease, ailment, or condition** | **Vectors for CAR-T cell modification** |
|---|---|---|
| 1 | solid tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 2 | anal tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 3 | rectal tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 4 | epithelial tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 5 | ovarian tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 6 | breast tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 7 | fallopian tube tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 8 | endometrial tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 9 | pancreatic tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 10 | colorectal tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 11 | lung tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 12 | gastrointestinal tumors | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 13 | melanoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 14 | multiple myeloma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 15 | acute lymphoblastic leukemia | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 16 | acute myelocytic leukemia | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 17 | non-Hodgkin lymphoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 18 | diffuse large b-cell lymphoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 19 | small-cell lung cancer | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 20 | neuroblastoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 21 | glioblastoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 22 | prostate cancer | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 23 | hemophilia | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 24 | sickle cell disease | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 25 | lupus erythematosus | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 26 | lupus nephritis | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 27 | myasthenia gravis | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 28 | autoimmune diseases | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 29 | soft tissue sarcoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 30 | osteosarcoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 31 | hepatocellular carcinoma | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 32 | graft versus host disease | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 33 | rheumatoid arthritis | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |
| 34 | impairments related to renal transplantation | mRNA, shRNA, siRNA, saRNA, SeekRNA, polymers, proteins and peptides, antibodies, viruses, including but not limited to lentivirus (LV), Adeno Associated Virus (AAV), labelling molecules, small molecules, Virus-like particles (VLP), transposon/transposase (sleeping beauty, TcBuster, PiggyBac), transcription activator-like effector nuclease (TALEN), zinc finger nucleases (ZFN), base editors, prime editors, programmable addition via site-specific targeting elements (PASTE), CRISPR/Cas, and CRISPR RNPs |

**Table 2**

| **Embodiment #** | **Vector for CAR-T cell modification** | **Diseases, ailments, or conditions** |
|---|---|---|
| 35 | mRNA | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 36 | shRNA | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 37 | siRNA | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 38 | saRNA | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 39 | SeekRNA | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 40 | polymers | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 41 | proteins | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 42 | peptides | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 43 | antibodies | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 44 | viruses | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 45 | Lentivirus (LV) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 46 | Adeno Associated Virus (AAV) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 47 | labelling molecules | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 48 | small molecules | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 49 | Virus-like particles (VLP) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 50 | transposon/transposase (sleeping beauty) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 51 | transposon/transposase (TcBuster) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 52 | transposon/transposase (PiggyBac) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 53 | transcription activator-like effector nuclease (TALEN) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 54 | zinc finger nucleases (ZFN) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 55 | base editors | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 56 | prime editors | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 57 | programmable addition via site-specific targeting elements (PASTE) | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 58 | CRISPR/Cas | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |
| 59 | CRISPR RNPs | solid tumors such as anal/rectal, epithelial, ovarian, breast, fallopian tube, endometrial, pancreatic, colorectal, lung, and/or gastrointestinal tumors; melanoma, multiple myeloma, acute lymphoblastic leukemia, acute myelocytic lymphoma, non-Hodgkin lymphoma, diffuse large b-cell lymphoma, small-cell lung cancer, neuroblastoma, glioblastoma, prostate cancer, hemophilia, sickle cell disease, lupus erythematosus, lupus nephritis, myasthenia gravis, autoimmune diseases, soft tissue sarcoma, osteosarcoma, hepatocellular carcinoma, graft versus host disease, rheumatoid arthritis, and impairments related to renal transplantation |

Embodiments 1 to 59 listed in Tables 1 and 2 do not only apply to modified CAR-T cells, but also to any other modified cell types that can be produced by the blood processing system.

Accordingly, a "modified cell" as used herein is a cell that has been produced by the blood processing system by use of the vectors of embodiments 35 to 59 (Table 2). Said modified cell is further provided for use in the treatment of a disease, ailment, or condition according to embodiments 1 to 34 (Table 1).

In an embodiment, the modified cells are modified Chimeric Antigen Receptor Natural Killer (CAR-NK) cells.

In a further embodiment, the modified cells are modified Chimeric Antigen Receptor Monocytes (CAR-M) cells.

In another embodiment, the modified cells are modified Engineered T-Cell Receptor (TCR) cells.

In a further embodiment, the modified cells are modified Engineered B-Cells.

In another embodiment, the modified cells are modified Tumor Infiltrating Lymphocytes (TIL) cells.

In a further embodiment, the modified cells are modified Induced Pluripotent Stem (iPSC) Cells.

In another embodiment, the modified cells are modified Invariant Natural Killer T (iNKT) cells.

In a further embodiment, the modified cells are modified Mesenchymal Stem (MSC) cells.

In another embodiment, the modified cells are modified Dendritic Cells.

In a further embodiment, the modified cells are modified Hematopoietic Stem Cells/Stem (CD34+) cells.

The modified cells can be produced and administered in a variety of environments by the blood processing system, with the modified cells being produced using any one of the preceding vectors and being administered in any one of the preceding applications or therapeutic treatments. By way of example, the blood processing system can produce and/or administer modified cells within a patient treatment room/facility (inpatient or outpatient). The blood processing system can produce and/or administer modified cells within a cell processing lab. The cell processing lab may be at a hospital facility, at a non-hospital facility, or a commercial production facility (centralized or decentralized). The blood processing system can produce and/or administer modified cells within a sterile manufacturing suite. The sterile manufacturing suite may be at a hospital facility, at a non-hospital facility, at an academic facility, or at commercial production facility (centralized or decentralized).

While the modified cells produced by this system are described as being used to treat particular conditions and patient groups, it should be understood that they may be applied to other conditions and/or patient groups, including subgroups of a described patient group (i.e., patients having the same characteristics that characterize a particular patient group, but additional characteristics that are not shared by all patients of that patient group), larger patient groups encompassing a described patient group (i.e., a patient group having broadly defined characteristics that include the characteristics that characterize a particular patient group) and entirely different patient groups (i.e., patients having characteristics that exclude them from a particular patient group). The modified cells may also be applied in various dosages, administrative regimes, and routes of administration without departing from the scope of the present disclosure.

It should be understood that the embodiments disclosed herein may be combined with each other in any imaginable combination.

### Aspects

Aspect 1. A cell selection chamber, comprising: an inlet; an outlet; a reservoir positioned between the inlet and the outlet, configured to allow for fluid flow from the inlet to the outlet, and containing a plurality of microbubbles; an upstream membrane positioned between the inlet and the reservoir; and a downstream membrane positioned between the outlet and the reservoir, wherein the upstream membrane and the downstream membrane each include a plurality of pores having a diameter less than a diameter of the microbubbles, and each of the microbubbles is at least partially coated with streptavidin.

Aspect 2. The cell selection chamber of Aspect 1, wherein the microbubbles are formed of silica.

Aspect 3. The cell selection chamber of any one of the preceding Aspects, wherein the pores each have a diameter of less than 50 microns.

Aspect 4. A blood processing system, comprising: a blood processing device including a pump system, a valve system, and a controller; and a fluid flow circuit configured to be mounted to the blood processing device and including a cell selection chamber, wherein the cell selection chamber includes an inlet, an outlet, a reservoir positioned between the inlet and the outlet, configured to allow for fluid flow from the inlet to the outlet, and containing a plurality of microbubbles, an upstream membrane positioned between the inlet and the reservoir, and a downstream membrane positioned between the outlet and the reservoir, the upstream membrane and the downstream membrane each include a plurality of pores having a diameter less than a diameter of the microbubbles, each of the microbubbles is at least partially coated with streptavidin, and the controller is programmed to control the pump system and the valve system during a blood processing procedure to convey a heterogeneous population of cells into the cell selection chamber so as to cause target cells of said heterogeneous population of cells to become bound to the microbubbles and to cause other cells of the heterogeneous population of cells to flow out of the cell selection chamber as unbound cells.

Aspect 5. The blood processing system of Aspect 4, wherein the microbubbles are formed of silica.

Aspect 6. The blood processing system of any one of Aspects 4-5, wherein the pores each have a diameter of less than 50 microns.

Aspect 7. The blood processing system of any one of Aspects 4-6, wherein the controller is further programmed to control the pump system and the valve system during the blood processing procedure to mix said heterogeneous population of cells with a biotinylated antibody cocktail including regions configured to bind to the target cells and not to said other cells of the heterogeneous population of cells.

Aspect 8. The blood processing system of any one of Aspects 4-6, wherein the fluid flow circuit includes a separation module positioned upstream of the cell selection chamber, and the controller is further programmed to control the pump system and the valve system during the blood processing procedure to convey blood or cellular blood components through the separation module so as to separate said heterogeneous population of cells from the blood or cellular blood components.

Aspect 9. The blood processing system of Aspect 8, wherein nucleated white blood cells are separated from red blood cells and platelets within the separation module, with the separated nucleated white blood cells becoming said heterogeneous population of cells.

Aspect 10. The blood processing system of any one of Aspects 8-9, wherein the controller is further programmed to control the pump system and the valve system during the blood processing procedure to mix said heterogeneous population of cells from the separation module with a biotinylated antibody additive including regions configured to bind to the target cells and not to said other cells of the heterogeneous population of cells.

Aspect 11. The blood processing system of any one of Aspects 4-10, wherein the fluid flow circuit includes a cell modification module positioned downstream of the cell selection chamber, and the controller is further programmed to control the pump system and the valve system during the blood processing procedure to convey the unbound cells from the cell selection chamber into the cell modification module so as to modify the unbound cells and created modified cells.

Aspect 12. The blood processing system of Aspect 11, wherein the controller is further programmed to control the pump system and the valve system during the blood processing procedure to convey the modified cells to a living recipient.

Aspect 13. The blood processing system of Aspect 11, wherein the controller is further programmed to control the pump system and the valve system during the blood processing procedure to convey the modified cells to a dose container.

Aspect 14. A method for selecting target cells from a heterogeneous population of cells, comprising: mixing a heterogeneous population of cells with a biotinylated antibody additive including regions configured to bind to the target cells and not to other cells of the heterogeneous population of cells; conveying the heterogeneous population of cells into a cell selection chamber via an inlet of the cell selection chamber; flowing the heterogeneous population of cells through an upstream membrane and into a reservoir of the cell selection chamber containing a plurality of microbubbles each at least partially coated with streptavidin so as to cause the target cells to become bound to the microbubbles; and flowing said other cells through a downstream membrane, out of the reservoir, and out of the cell selection chamber as unbound cells via an outlet of the cell selection chamber.

Aspect 15. The method of Aspect 14, wherein the microbubbles are formed of silica.

Aspect 16. The method of any one of Aspects 14-15, wherein the pores each have a diameter of less than 50 microns.

Aspect 17. The method of any one of Aspects 14-16, further comprising separating said heterogeneous population of cells from blood or cellular blood components.

Aspect 18. The method of any one of Aspects 14-17, further comprising modifying the unbound cells so as to create modified cells.

Aspect 19. The method of Aspect 18, further comprising conveying the modified cells to a living recipient.

Aspect 20. The method of Aspect 18, further comprising conveying the modified cells to a dose container.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A cell selection chamber (76'), comprising:
an inlet (88);
an outlet (90);
a reservoir (92) positioned between the inlet (88) and the outlet (90), configured to allow for fluid flow from the inlet (88) to the outlet (90), and containing a plurality of microbubbles (98);
an upstream membrane (94) positioned between the inlet (88) and the reservoir (92); and
a downstream membrane (96) positioned between the outlet (90) and the reservoir (92), wherein
the upstream membrane (94) and the downstream membrane (96) each include a plurality of pores having a diameter less than a diameter of the microbubbles (98), and
each of the microbubbles (98) is at least partially coated with streptavidin.

2. A blood processing system (21), comprising:
a blood processing device (20) including a pump system (30, 32, 54, 56, 58, 60), a valve system (V1-V21), and a controller (16); and
a fluid flow circuit (10) configured to be mounted to the blood processing device (20) and including the cell selection chamber (76') of claim 1, wherein the controller (16) is programmed to control the pump system (30, 32, 54, 56, 58, 60) and the valve system (V1-V21) during a blood processing procedure to convey a heterogeneous population of cells into the cell selection chamber (76') so as to cause target cells of said heterogeneous population of cells to become bound to the microbubbles (98) and to cause other cells of the heterogeneous population of cells to flow out of the cell selection chamber (76') as unbound cells.

3. The blood processing system (21) of claim 2, wherein the controller (16) is further programmed to control the pump system (30, 32, 54, 56, 58, 60) and the valve system (V1-V21) during the blood processing procedure to mix said heterogeneous population of cells with a biotinylated antibody cocktail including regions configured to bind to the target cells and not to said other cells of the heterogeneous population of cells.

4. The blood processing system (21) of claim 2, wherein
the fluid flow circuit (10) includes a separation module (62) positioned upstream of the cell selection chamber (76'), and
the controller (16) is further programmed to control the pump system (30, 32, 54, 56, 58, 60) and the valve system (V1-V21) during the blood processing procedure to convey blood or cellular blood components through the separation module (62) so as to separate said heterogeneous population of cells from the blood or cellular blood components.

5. The blood processing system (21) of claim 4, wherein nucleated white blood cells are separated from red blood cells and platelets within the separation module (62), with the separated nucleated white blood cells becoming said heterogeneous population of cells.

6. The blood processing system (21) of any one of claims 4-5, wherein the controller (16) is further programmed to control the pump system (30, 32, 54, 56, 58, 60) and the valve system (V1-V21) during the blood processing procedure to mix said heterogeneous population of cells from the separation module (62) with a biotinylated antibody additive including regions configured to bind to the target cells and not to said other cells of the heterogeneous population of cells.

7. The blood processing system (21) of any one of claims 2-6, wherein
the fluid flow circuit (10) includes a cell modification module positioned downstream of the cell selection chamber (76'), and
the controller (16) is further programmed to control the pump system (30, 32, 54, 56, 58, 60) and the valve system (V1-V21) during the blood processing procedure to convey the unbound cells from the cell selection chamber (76') into the cell modification module so as to modify the unbound cells and created modified cells.

8. The blood processing system (21) of claim 7, wherein the controller (16) is further programmed to control the pump system (30, 32, 54, 56, 58, 60) and the valve system (V1-V21) during the blood processing procedure to convey the modified cells to a living recipient.

9. The blood processing system (21) of claim 7, wherein the controller (16) is further programmed to control the pump system (30, 32, 54, 56, 58, 60) and the valve system (V1-V21) during the blood processing procedure to convey the modified cells to a dose container (50).

10. A method for selecting target cells from a heterogeneous population of cells, comprising:
mixing a heterogeneous population of cells with a biotinylated antibody additive including regions configured to bind to the target cells and not to other cells of the heterogeneous population of cells;
conveying the heterogeneous population of cells into a cell selection chamber (76') via an inlet (88) of the cell selection chamber (76');
flowing the heterogeneous population of cells through an upstream membrane (94) and into a reservoir (92) of the cell selection chamber (76') containing a plurality of microbubbles (98) each at least partially coated with streptavidin so as to cause the target cells to become bound to the microbubbles; and
flowing said other cells through a downstream membrane (96), out of the reservoir (92), and out of the cell selection chamber (76') as unbound cells via an outlet (90) of the cell selection chamber (76').

11. The cell selection chamber (76') of claim 1 or the blood processing system (21) of any one of claims 2-9 or the method of claim 10, wherein the microbubbles (98) are formed of silica.

12. The cell selection chamber (76') of claim 1 or the blood processing system (21) of any one of claims 2-9 or the method of any one of claims 10-11, wherein the pores each have a diameter of less than 50 microns.

13. The method of any one of claims 10-12, further comprising separating said heterogeneous population of cells from blood or cellular blood components.

14. The method of any one of claims 10-13, further comprising modifying the unbound cells so as to create modified cells.

15. The method of claim 14, further comprising conveying the modified cells to a dose container.
